# EUROPEAN PATENT APPLICATION

(11) **EP 4 775 673 A1**
(43) Date of publication of application: **15.07.2026**
(21) Application number: 24861506.4
(22) Date of filing: 22.04.2024
(51) Int. Cl.: C12N 9/02, C12N 9/00, C12N 15/53, C12N 15/52, C12P 17/02

(54) **METHOD AND BIOMATERIAL FOR TOTAL BIOSYNTHESIS OF PACLITAXEL PRECURSOR SUBSTANCE BACCATIN III, AND USE**

(30) Priority: 04.09.2023 CN 202311137424
(71) Applicant: Agricultural Genomics Institute at Shenzhen, Chinese Academy of Agricultural Sciences (Shenzhen Branch, Guangdong Laboratory for, Shenzhen, Guangdong 518124 (CN); Agricultural Genomics Institute, Chinese Academy of Agricultural Sciences, Shenzhen, Guangdong 518124 (CN)
(72) Inventor: YAN, Jianbin, Shenzhen, Guangdong 518124 (CN); JIANG, Bin, Shenzhen, Guangdong 518124 (CN); SUN, Yaping, Shenzhen, Guangdong 518124 (CN); HUANG, Sanwen, Shenzhen, Guangdong 518124 (CN)
(74) Representative: Murgitroyd & Company
(86) International application number: PCT/CN2024/089090
(87) International publication number: WO 2025/050656

(57) **Abstract**

A method and a biomaterial for the total biosynthesis of a key paclitaxel precursor substance baccatin III, and the use. The biological enzyme composition used in the method comprises taxane 9α-hydroxylase (T9αH)and taxane C4-C20 oxetane-forming enzyme (TOT). The core ingredients of the biosynthetic pathway of baccatin III are identified. and an artificial synthesis route for baccatin III is constructed, which pave the way for the efficient development of a green and low-carbon production pathway for taxane products such as paclitaxel by means of synthetic biology.

## Description

### TECHNICAL FIELD

The disclosure relates to the technical field of plant metabolic biology and synthetic biology, and particularly relating to a method for biological total synthesis of paclitaxel key precursor substance baccatin III, biological material, and use thereof.

### BACKGROUND

Paclitaxel is a diterpenoid alkaloid compound derived from *Taxus chinensis* plant, with a structural formula as shown in Formula I. It has been widely used clinically in the treatment of breast cancer, ovarian cancer, some head and neck cancers, and lung cancer. At present, a semi-chemical synthesis method is generally used to produce paclitaxel, i.e., firstly extracting the natural precursor substance baccatin III or 10-deacetylbaccatin III (10-DAB) from the leaves, and then chemically synthesizing paclitaxel.

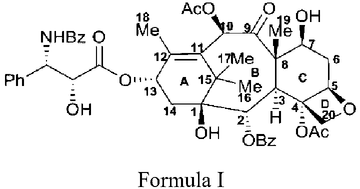

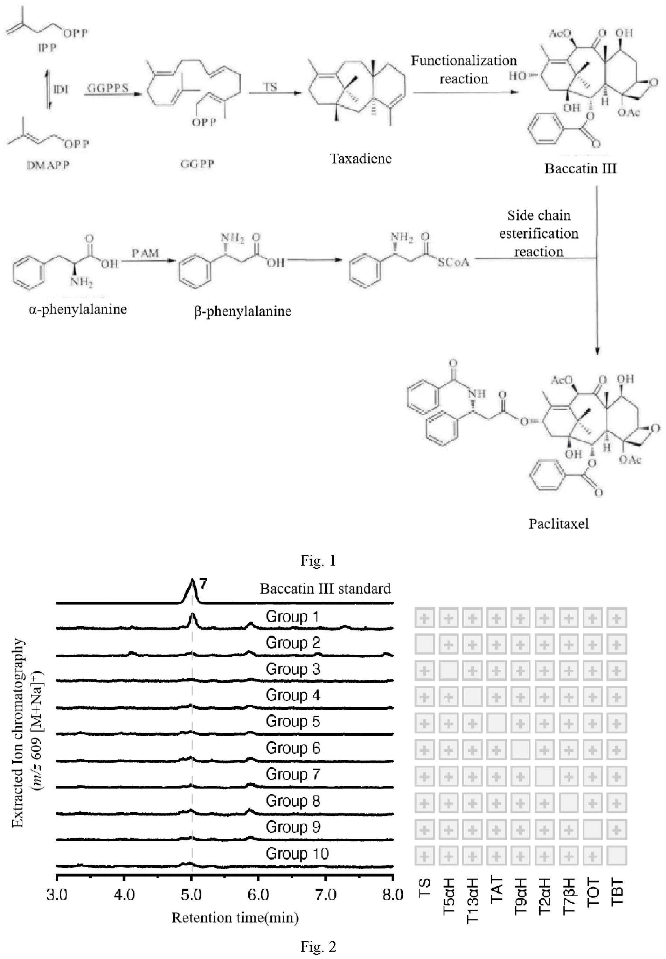

Since the structure of paclitaxel was first discovered in 1971, elucidating its biosynthetic pathway has been a focus in the field of natural product research. As shown in Fig. 1, the biosynthetic pathway of paclitaxel starts from the linear coupling of isoprenyl precursors (IPP and DMAPP) with GGPP to form taxadiene. Taxadiene undergoes a series of oxidation, acylation, and epoxidation to form the key intermediate baccatin III. Baccatin III is finally conjugated to the C13 side chain to generate paclitaxel. It is inferred, according to the structure of the intermediate, that the synthesis of paclitaxel requires about 19 enzymatic reactions, however, 4 oxidases and 1 mutase still have not been identified, they are respectively C9 hydroxylase (Taxane 9α hydroxylase, T9αH), C9 oxidase (Taxane 9α dioxygenase, T9αO), C1 hydroxylase (Taxane 1β hydroxylase, T1βH), C4-C20 epoxidase (C4β, C20-epoxidase, EPOX) and oxomutase (OXM). Since the key enzymes for the formation of C4-C20 oxygen-including heterocycle and oxidation of C9 are still unclear, the biosynthesis of paclitaxel intermediate baccatin III is impossible, and a complete paclitaxel biosynthetic pathway has been lacking for a long time.

### SUMMARY

In view of this, the disclosure provides a method for biological total synthesis of paclitaxel key precursor substance baccatin III, biological material and use thereof. This disclosure successfully identified the gene of taxane C4-C20 oxetanase (TOT) and the gene of taxane 9α-hydroxylase (T9αH), and identified the core components of the baccatin III biosynthetic pathway, paving the way for the efficient development of a green and low-carbon production pathway for taxane products (such as paclitaxel) through synthetic biology.

In order to achieve the above object, the disclosure provides the following technical solutions:
The disclosure provides a bioenzyme composition, which includes taxane 9α-hydroxylase (T9αH) and taxane C4-C20 oxetanase (TOT);
the amino acid sequence of T9αH includes at least one of the following (a1) to (a4):
(a1) at least one of the amino acid sequences represented by SEQ ID NOs: 1-14;
(a2) a fusion amino acid sequence obtained by connecting a tag to N-terminus and/or C-terminus of at least one of the amino acid sequences represented by SEQ ID NOs: 1-14;
(a3) an amino acid sequence having the same function obtained by substitution and/or deletion and/or addition of one or more amino acid residues in at least one of the amino acid sequences represented by SEQ ID NOs: 1-14;
(a4) an amino acid sequence having at least 60% identity with at least one of the amino acid sequences of SEQ ID NOs: 1-14 and having the same function;
the amino acid sequence of TOT includes at least one of the following (b1) to (b4):
   (b1) at least one of the amino acid sequences represented by SEQ ID NOs: 29-52;
   (b2) a fusion amino acid sequence obtained by connecting a tag to N-terminus and/or C-terminus of at least one of the amino acid sequences represented by SEQ ID NOs: 29-52;
   (b3) an amino acid sequence having the same function obtained by substitution and/or deletion and/or addition of one or more amino acid residues in at least one of the amino acid sequences represented by SEQ ID NOs: 29-52;
   (b4) an amino acid sequence having at least 60% identical with at least one of the amino acid sequences of SEQ ID NOs: 29-52 and having the same function.

In the above (a4) or (b4), the amino acid sequence with at least 60% identity is exemplified by amino acid sequences with at least 60%, 65%, 70%, 75%, 80%, 85%, 90%, 91%, 92%, 93%, 94%, 95%, 96%, 97%, 98% or 99% identity.

In particular embodiments of the disclosure, the bioenzyme composition includes taxane 9α-hydroxylase (T9αH) and taxane C4-C20 oxetanase (TOT);
the amino acid sequence of T9αH includes at least one of the following (a1) to (a4):
(a1) an amino acid sequence represented by SEQ ID NO: 1;
(a2) a fusion amino acid sequence obtained by connecting a tag to N-terminus and/or C-terminus of the amino acid sequence represented by SEQ ID NO: 1;
(a3) an amino acid sequence having the same function obtained by substitution and/or deletion and/or addition of one or more amino acid residues in the amino acid sequence represented by SEQ ID NO: 1;
(a4) an amino acid sequence having at least 60% identity with the amino acid sequence of SEQ ID NO: 1 and having the same function;
the amino acid sequence of the TOT includes at least one of the following (b1) to (b4):
   (b1) an amino acid sequence represented by SEQ ID NO: 29;
   (b2) a fusion amino acid sequence obtained by connecting a tag to N-terminus and/or C-terminus of the amino acid sequence represented by SEQ ID NO: 29;
   (b3) an amino acid sequence having the same function obtained by substitution and/or deletion and/or addition of one or more amino acid residues in the amino acid sequence represented by SEQ ID NO: 29;
   (b4) an amino acid sequence having at least 60% identity with the amino acid sequence of SEQ ID NO: 29 and having the same function.

In particular embodiments of the disclosure, in (a4), the amino acid sequence having at least 60% identity with and the same function as the amino acid sequence represented by SEQ ID NO: 1 includes at least one of the amino acid sequences represented by SEQ ID NO: 2-14; and/or in (b4), the amino acid sequence having at least 60% identity with and the same function as the amino acid sequence represented by SEQ ID NO: 29 includes at least one of the amino acid sequences represented by SEQ ID NO: 30-52.

In particular embodiments of the disclosure, the bioenzyme composition includes a paclitaxel T9αH enzyme of the amino acid sequence represented by SEQ ID NO: 1 and a TOT enzyme of any one of the amino acid sequences represented by SEQ ID NOs: 30-52.

In particular embodiments of the disclosure, the bioenzyme composition includes a taxane T9αH enzyme of any one of the amino acid sequences represented by SEQ ID NOs: 2-14 and a TOT enzyme of the amino acid sequence represented by SEQ ID NO: 29.

In particular embodiments of the disclosure, the bioenzyme composition includes a taxane T9αH enzyme of any one of the amino acid sequences represented by SEQ ID NOs: 2-14 and a TOT enzyme of any one of the amino acid sequences represented by SEQ ID NOs: 30-52.

Preferably, the bioenzyme composition includes a combination of the paclitaxel T9αH enzyme of the amino acid sequence represented by SEQ ID NO: 1 and a TOT enzyme of the amino acid sequence represented by SEQ ID NO: 29.

In particular embodiments of the disclosure, the sequence similarity of the above 14 amino acid sequences of T9αH (SEQ ID NOs: 1-14) is 63.15-100%, with conserved domains/sites consisting of conserved PSRF motif (amino acids 423-426), highly conserved heme-binding PFG element (amino acids 439-441), ETMR salt bridge (amino acids 369-372), EXXR motif, and critical cysteine (amino acid 447); the key amino acid sites of the mutant include: L75, G128, P129, F132, V136, V142, L228, T231, L232, F305, H308, A309, D312, T313, S316, P376, A377, F378, G379, S380, F381, R382, M402, F485, P486, and P487.

In particular embodiments of the disclosure, the amino acid sequence in b3) is a mutant of the amino acid sequence as shown in b1), the mutant includes at least one of: V237A, P167A, Q187A, Q6A, V129A, I172A, S314A, V319A, Q381A, E313A, V205A, R50A, S500A, P428A, H249A, S310A, K259A, R269A, M177A, D361A, V382A, I347A, V371A, G448A, P454AA, P490A, I285A, T410A, T231A, K360A, G455A, K94A, etc.

The research of the disclosure found that in the biosynthetic pathway of baccatin III, GGPP is converted into taxadiene under the catalysis of taxadiene synthase (TS); subsequently, taxadiene is modified by the coordinated catalysis of six endoplasmic reticulum-related p450 enzymes, including taxane 5α-hydroxylase (T5αH), taxane 2α-hydroxylase (T2αH), taxane 7β-hydroxylase (T7βH), taxane 9α-hydroxylase (T9αH), taxane 13α-hydroxylase (T13αH) and taxane C4-C20 oxetanase (TOT), and is finally converted into baccatin III under the catalysis of two cytoplasmic acyltransferases (taxane 5α-acetyltransferase (TAT) and taxane 2α-O-benzoyl transferase (TBT)).

In one embodiment of the disclosure, when the starting material is geranylgeranyl pyrophosphate (GGPP), and baccatin III is synthesized, the bioenzyme composition of the disclosure further includes at least one of: taxadiene synthase (TS), taxane 5α-hydroxylase (T5αH), taxane 13α-hydroxylase (T13αH), taxane 2α-hydroxylase (T2αH), taxane 7β-hydroxylase (T7βH), taxane 5α-acetyl transferase (TAT), and taxane 2α-O-benzoyl transferase (TBT).

Preferably, the bioenzyme composition includes all the aforementioned bioenzymes: taxadiene synthase (TS), taxane 5α-hydroxylase (T5αH), taxane 13α-hydroxylase (T13αH), taxane 2α-hydroxylase (T2αH), taxane 7β-hydroxylase (T7βH), taxane 5α-acetyl transferase (TAT), and taxane 2α-O-benzoyl transferase (TBT).

In some embodiments, the natural amino acid sequences of the above various bioenzymes may be variant sequences derived from different species and/or plants of *Taxus.* The artificial amino acid sequences of the above various bioenzymes may be variant sequences obtained by appropriate modification of the natural amino acid sequences, and the modifications include but are not limited to appropriate amino acid substitutions/additions/deletions, truncation of the N-terminal amino acid, codon optimization suitable for host cell preference, addition and fusion of tags, etc., which do not affect the biological activity of the target protein.

The above bioenzyme may be a bioenzyme being removed the signal peptide, or may be a bioenzyme including the signal peptide.

In another embodiment of the disclosure, when the starting material is geranylgeranyl pyrophosphate (GGPP), and paclitaxel as the downstream product of baccatin III is synthesized, in addition to the above T9αH and TOT, and in addition to at least one of: TS, T5αH, T13αH, T2αH, T7βH, TAT and TBT, the bioenzyme composition further includes at least one of: phenylalanyl-CoA ligase (PCL), phenylalanine aminomutase (PAM), baccatin III (3-amino-3-phenylpropanoyl transferase, BAPT), cytochrome P450 hydroxylase (T2'αH), and 3'-N-debenzoyl-2'-deoxytaxol-N-benzoyl transferase (DBTNPT).

Preferably, the bioenzyme composition includes all the aforementioned types of bioenzymes: TS T5αH, T13αH, T2αH, T7βH, TAT**,** TBT, T9αH, TOT, Phenylalanyl CoA ligase, phenylalanine aminomutase, baccatin III 3-amino-3-phenylpropanoyl transferase, cytochrome P450 hydroxylase, 3'-N-debenzoyl-2'-deoxytaxol-N-benzoyl transferase.

In another embodiment of the disclosure, when the starting material is the precursor of GGPP as isopentenyl pyrophosphate (IPP) and/or dimethylallyl pyrophosphate (DMAPP)) for synthesizing baccatin III, in this embodiment of the disclosure, the bioenzyme composition further includes geranylgeranyl pyrophosphate synthase (GGPPS). The precursor molecules IPP and DMAPP for synthesizing GGPP can be provided by the host cells themselves.

The disclosure provides a nucleic acid molecule composition encoding the above bioenzyme composition, which includes a nucleic acid molecule encoding T9αH and a nucleic acid molecule encoding TOT.

The nucleotide sequence of the nucleic acid molecule encoding T9αH includes at least one of the following sequences:
(c1) at least one of the nucleotide sequences represented by SEQ ID NOs: 15-28;
(c2) a complementary sequence, degenerate sequence or homologous sequence of at least one of the nucleotide sequences represented by SEQ ID NOs: 15-28, where a homologous sequence is a nucleotide sequence having at least 75% identity with the nucleotide sequence represented by any one of SEQ ID NOs: 15-28; a nucleotide sequence having at least 75% identity is exemplarily a nucleotide sequence having at least 75%, 80%, 85%, 90%, 91%, 92%, 93%, 94%, 95%, 96%, 97%, 98% or 99% identity.
(c3) a nucleotide sequence hybridizing with the nucleotide sequence as shown in (c1) or (c2) under stringent conditions and being capable of encoding a protein with the same function;

The nucleotide sequence of the nucleic acid molecule encoding POT includes at least one of the following sequences:
(d1) at least one of the nucleotide sequences represented by SEQ ID NOs: 53-76;
(d2) a complementary sequence, degenerate sequence or homologous sequence of at least one of the nucleotide sequences represented by SEQ ID NOs: 53-76, where a homologous sequence is a nucleotide sequence having at least 74% identity with the nucleotide sequence represented by any one of SEQ ID NOs: 53-76; a nucleotide sequence having at least 74% identity is exemplarily a nucleotide sequence having at least 74%, 75%, 80%, 85%, 90%, 91%, 92%, 93%, 94%, 95%, 96%, 97%, 98% or 99% identity; and
(d3) a nucleotide sequence hybridizing with the nucleotide sequence as shown in (d1) or (d2) under stringent conditions and being capable of encoding a protein with the same function.

In particular embodiments of the disclosure, the nucleic acid molecule composition of the above bioenzyme composition includes a nucleic acid molecule encoding T9αH and a nucleic acid molecule encoding TOT; where
the nucleotide sequence of the nucleic acid molecule encoding T9αH includes at least one of the following sequences:
(c1) a nucleotide sequence represented by SEQ ID NO: 15;
(c2) a complementary sequence, degenerate sequence, or homologous sequence of the nucleotide sequence represented by SEQ ID NO: 15, where the homologous sequence is a nucleotide sequence having at least 75% identity with the nucleotide sequence represented by SEQ ID NO: 15;
(c3) a nucleotide sequence hybridizing with the nucleotide sequence as shown in (c1) or (c2) under strict conditions and being capable of encoding a protein with the same function;
the nucleotide sequence of the nucleic acid molecule encoding TOT includes at least one of the following sequences:
   (d1) a nucleotide sequence represented by SEQ ID NO: 53;
   (d2) a complementary sequence, degenerate sequence, or homologous sequence of the nucleotide sequence represented by SEQ ID NO: 53, where the homologous sequence is a nucleotide sequence having at least 74% identity with the nucleotide sequence represented by SEQ ID NO: 53;
   (d3) a nucleotide sequence hybridizing with the nucleotide sequence as shown in (d1) or (d2) under strict conditions and being capable of encoding a protein with the same function.

In particular embodiments of the disclosure, in (c2), the homologous sequence includes at least one of the nucleotide sequences represented by SEQ ID NOs: 16-28; and/or, in (d2), the homologous sequence includes at least one of the nucleotide sequences represented by SEQ ID NOs: 54-76.

In particular embodiments of the disclosure, the nucleic acid molecule composition encoding the above bioenzyme composition includes: a nucleotide sequence encoding T9αH represented by any one of SEQ ID NOs: 16-28, and a nucleotide sequence encoding TOT as represented by SEQ ID NO: 53.

In particular embodiments of the disclosure, the nucleic acid molecule composition encoding the above bioenzyme composition includes: a nucleotide sequence encoding T9αH as represented by SEQ ID NO: 15, and a nucleotide sequence encoding TOT represented by any one of SEQ ID NOs: 54-76.

In particular embodiments of the disclosure, the nucleic acid molecule composition encoding the above bioenzyme composition includes: a nucleotide sequence encoding T9αH represented by any one of SEQ ID NOs: 16-28, and a nucleotide sequence encoding TOT represented by any one of SEQ ID NOs: 54-76.

Preferably, the nucleic acid molecule encoding T9αH includes: a nucleotide sequence encoding any one of the amino acid sequences represented by SEQ ID NOs: 1-14. Preferably, the nucleic acid molecule encoding T9αH includes: any of the nucleotide sequences represented by SEQ ID NOs: 15-28.

More preferably, the nucleic acid molecule encoding T9αH is a nucleotide sequence encoding the amino acid sequence represented by SEQ ID NO: 1. Still more preferably, the nucleic acid molecule encoding T9αH includes a nucleotide sequence represented by SEQ ID NO: 15.

Preferably, the nucleic acid molecule encoding TOT includes: a nucleotide sequence encoding any one of the amino acid sequences represented by SEQ ID NOs: 29-52. Preferably, the nucleic acid molecules encoding TOT include: any one of the nucleotide sequences represented by SEQ ID NOs: 53-76.

More preferably, the nucleic acid molecule encoding TOT is a nucleotide sequence encoding the amino acid sequence represented by SEQ ID NO: 29. Still more preferably, the nucleic acid molecule encoding TOT includes the nucleotide sequence represented by SEQ ID NO: 53.

Preferably, the nucleic acid molecule composition encoding the above bioenzyme composition includes: a nucleotide sequence encoding the amino acid sequence represented by SEQ ID NO: 1, and a nucleotide sequence encoding the amino acid sequence represented by SEQ ID NO: 29.

More preferably, the nucleic acid molecule composition encoding the above bioenzyme composition includes: a nucleotide sequence encoding T9αH represented by SEQ ID NO: 15, and a nucleotide sequence encoding TOT represented by SEQ ID NO: 53.

In one embodiment of the disclosure, when baccatin III is synthesized from GGPP, the above nucleic acid molecule composition further includes a nucleic acid molecule encoding at least one of the following bioenzymes: taxadiene synthase (TS), taxane 5α-hydroxylase (T5αH), taxane 13α-hydroxylase (T13αH), taxane 2α-hydroxylase (T2αH), taxane 7β-hydroxylase (T7βH), taxane 5α-acetyl transferase (TAT), and taxane 2α-O-benzoyl transferase (TBT).

Preferably, the nucleic acid molecule composition includes a nucleic acid molecule encoding all types of the following bioenzymes: taxadiene synthase (TS), taxane 5α-hydroxylase (T5αH), taxane 13α-hydroxylase (T13αH), taxane 2α-hydroxylase (T2αH), taxane 7β-hydroxylase (T7βH), taxane 5α-acetyl transferase (TAT), and taxane 2α-O-benzoyl transferase (TBT).

In some embodiments, the natural nucleotide sequences of the nucleic acid molecule related to the disclosure may be a variant sequence derived from different species and/or plants of *Taxus,* and its artificial nucleotide sequence may be variant sequences obtained by appropriate modification of the natural nucleotide sequence, and the modifications include but are not limited to appropriate nucleotide substitutions/additions/deletions, truncation of the N-terminal nucleotide, codon optimization suitable for host cell preference, addition and fusion of tags, etc., which do not affect the biological activity of the target protein.

In another embodiment of the disclosure, when paclitaxel as a downstream product of baccatin III is synthesized from GGPP, the nucleic acid molecule composition further includes a nucleic acid molecule encoding at least one of the following bioenzymes: phenylalanyl-CoA ligase (PCL), phenylalanine aminomutase (PAM), baccatin III 3-amino-3-phenylpropanoyl transferase (BAPT), cytochrome P450 hydroxylase (T2'αH), and 3'-N-debenzoyl-2'-deoxytaxol-N-benzoyl transferase (DBTNPT).

Preferably, the nucleic acid molecule composition includes a nucleic acid molecule encoding all types of the following bioenzymes: phenylalanyl-CoA ligase (PCL), phenylalanine aminomutase (PAM), baccatin III 3-amino-3-phenylpropanoyl transferase (BAPT), cytochrome P450 hydroxylase (T2'αH), and 3'-N-debenzoyl-2'-deoxytaxol-N-benzoyl transferase (DBTNPT).

In an embodiment of the disclosure, when baccatin III is synthesized from IPP and/or DMAPP (the precursors of GGPP), the nucleic acid molecule composition further includes a nucleic acid molecule encoding geranylgeranyl pyrophosphate synthase (GGPPS), and/or IPP isomerase.

The nucleic acid molecules related to this disclosure can be obtained from a variety of sources. In some embodiments, part of the nucleic acid molecules are provided by the host cell itself, for example, the nucleotide molecules required for the synthesis of GGPP can be provided by the host cell itself, and the synthesis of taxadiene and/or taxadiene-5α-ol can be provided by the transformed host cells that have been disclosed, for example, the corresponding contents disclosed in the following literatures: Ajikumar PK, Xiao WH, Tyo KE, et al. Isoprenoid pathway optimization for Taxol precursor overproduction in Escherichia coli [J]. Science, 2010, 330 (6000): 70-74; Zhou K, Qiao K, Edgar S, et al. Distributing a metabolic pathway among a microbial consortium enhances production of natural products [J]. Nature Biotechnology, 2015, doi: 10. 1038/nbt. 3095.

Optionally, the nucleic acid molecule is synthetic and/or isolated.

The disclosure provides a biomaterial, where the biomaterial is any one of the following (e1) to (e3):
(e1) an expression cassette including the above nucleic acid molecule composition or a composition thereof;
(e2) a vector or a composition thereof, including the above nucleic acid molecule composition or the expression cassette of (e1) or a composition thereof;
(e3) a host cell or a composition thereof, including at least one of: the above nucleic acid molecule composition, the expression cassette of (e1) or a composition thereof, and the vector of (e2) or a composition thereof.

In the present embodiment, (e 1) is an expression cassette in which all nucleic acid molecules are expressed; or a composition of multiple expression cassettes in which all nucleic acid molecules are co-expressed.

In the present embodiment, (e2) is a vector in which all nucleic acid molecules are expressed, or a composition of multiple vectors in which all nucleic acid molecules are co-expressed.

In the present embodiment, (e3) is a host cell in which all nucleic acid molecules are expressed; or a composition of multiple host cells in which all nucleic acid molecules are co-expressed through co-culture and/or multi-stage culture.

In one embodiment of the disclosure, the biological material is an expression cassette or a composition thereof.

In an embodiment of the disclosure, when the nucleic acid molecule composition includes nucleic acid molecules encoding two bioenzymes: T9αH and TOT, it can be prepared into an expression cassette to express the T9αH gene and the TOT gene in one expression cassette; or it can be prepared into a composition of multiple expression cassettes to express the T9αH gene and the TOT gene in two expression cassettes, respectively.

In an embodiment of the disclosure, when the nucleic acid molecule composition includes a nucleic acid molecules encoding two bioenzymes: T9αH and TOT, and further includes a nucleic acid molecule encoding at least one of: TS, T5αH, T13αH, T2αH, T7βH, TAT and TBT, it can be prepared into an expression cassette to express all nucleic acid molecules in one expression cassette; alternatively, it can be prepared into a composition of multiple expression cassettes, to express all nucleic acid molecules in multiple expression cassettes, for example, to express one or more nucleic acid molecules in one expression cassette, as long as co-expression of all nucleic acid molecules can be achieved.

In an embodiment of the disclosure, the expression cassette further includes a regulatory element, and the regulatory element includes at least one of: a promoter, an enhancer, a leader sequence, a transposon, a terminator, and a marker gene.

In an embodiment of the disclosure, the expression cassette includes a native promoter and/or a heterologous promoter.

In an embodiment of the disclosure, the choice of an operably linked heterologous promoter may depend on a number of factors, for example, desired timing, localization, and expression pattern, and responsiveness to particular biotic or abiotic stimuli. Heterologous promoters include but are not limited to: inducible promoter, constitutive promoter, tissue-specific promoter, wound-inducible promoter, and chemically-regulated promoter, etc. For example, the heterologous promoter includes but is not limited to: Trc, T5, Tac, T7, T7lac, Sp6, araBAD, trp, lac, Ptac, pL, T3, GAL1, GAL10, MET17, CUP1, AOX1, polyhedrin, CMV, EF1A, EFS, CAG, PGK1, CBh, SFFV, MSCV, SV40, mPGK, hPGK, UBC, human beta actin, Actin, CaMV35S, TEF1, GPD, GDS, Ubi, ADH1, GAP, actin5C, Polyubiquitin, a1tubulin, TRE, TRE3G, UAS, Ac5, etc.

In an embodiment of the disclosure, the expression cassette further includes an expression cassette of modified nucleic acid sequences of the enzymes in (a)-(d), where a modification may be addition of a 5'-leader sequence to enhance the effect of translation and expression of a sequence; a modification may also be addition of a regulatory element, or a modification to the promoter, etc.

The precise nature of regulatory sequences for gene expression may vary between species or cell types, but generally will include 5'-nontranscribed and 5'-nontranslated sequences for the initiation of transcription and translation, e.g., the TATA box, capping sequence, CAAT sequence, etc. In particular, such 5'-nontranscribed regulatory sequences will include a promoter region including a promoter sequence that controls the transcriptional control of an operably linked gene. Regulatory sequences may further include enhancer sequences or desired upstream activator sequences. The expression cassettes of the disclosure may optionally include a 5'-leader or signal sequence.

In an embodiment of the disclosure, the expression of a nucleic acid molecule can be regulated by manipulating the copy number of a gene or operon in a cell.

In some embodiments, the expression of nucleic acid molecules can be regulated by manipulating the order of nucleic acid molecules within a module.

In some embodiments, expression of nucleic acid molecules is regulated by integrating one or more nucleic acid molecules or operons into a chromosome.

In another embodiment of the disclosure, the biological material is a carrier or a composition thereof.

In some embodiments, one or more nucleic acid molecules related to the disclosure are expressed in an expression vector. As used herein, "vector" can be any of a number of nucleic acids into which one or more desired sequences can be inserted by restriction enzyme digestion and ligation for transport in a different genetic environment or for expression in a host cell. Generally, a vector is either consists of DNA, or consists of RNA.

In an embodiment of the disclosure, the vector includes a plasmid, a chloroplast, a viral vector, a phage, a phagemid, a cosmid, a fosmid, a bacteriophage, or an artificial chromosome; and optionally, the viral vector includes an adenoviral vector, a retroviral vector or an adeno-associated viral vector; and optionally, the vector includes a bacterial artificial chromosome (BAC), a plasmid, a bacteriophage P1-derived vector (PAC), a yeast artificial chromosome (YAC) or a mammalian artificial chromosome (MAC). For example, the vector includes pFastBac1, pYES2, pYES2.1, pESC-Ura, pESC-Trp, pESC-Leu, pESC-His, pGEX2T, pTAex3, pUSA, pYMB0, pHT43, pET28b, pIJ702, pUCP19, pYMB03, pHT43, and pEAQ, etc.

A cloning vector is capable of autonomous replication or integration into the host cell genome and is also characterized by having one or more restriction endonuclease sites at which the vector can be cut in a defined manner, and the desired DNA sequence can be ligated into the vector, so that the new plasmid retains its ability to replicate in the host cell. In the case of a plasmid present in a host cell, replication of the desired sequence may occur multiple times as the plasmid increases its copy number in a host cell (such as a bacterial host), or may occur only once in a host before the host reproduces by mitosis. In the case of a bacteriophage present in a host cell, the replication can occur actively during the lytic phase or passively during the lysogenic phase.

A desired DNA sequence can be inserted into an expression vector by restriction enzyme digestion and ligation, so that it is operably linked to the regulatory sequence and can be expressed as RNA transcripts. A vector may also contain one or more marker sequences suitable for identifying whether a cell is transformed or transfected with the vector. A marker includes, for example, a gene encoding a protein increasing or decreasing resistance or sensitivity to an antibiotic or other compounds, a gene encoding an enzyme whose activity can be detected by standard methods known in the art (e.g., β-galactosidase, luciferase, or alkaline phosphatase), and a gene that have a visible effect on the phenotype of a transformed or transfected cell, host, colony or plaque (e.g., green fluorescent protein). Preferred vectors are those capable of autonomous replication and expression of the product of a structural gene present in the DNA fragment to which they are operably linked.

In some embodiments, the nucleic acid molecule compositions of the disclosure can be stably integrated into the genome (including the chloroplast genome) of a host cell.

In other embodiments, modified viruses and/or modified viral nucleic acids are applied to a plant or parts thereof by microinjection, microparticle bombardment, viral vector infection, or by spraying, irrigation, dusting, etc., so that the nucleic acid molecules are not stably integrated into the genome of the host cell, causing the host or host cell to transiently express the target gene.

In an embodiment of the disclosure, when the nucleic acid molecule composition includes a nucleic acid molecule encoding two bioenzymes: T9αH and TOT, it can be prepared into one vector to express T9αH nucleic acid molecules and TOT nucleic acid molecules in one vector; or it can be prepared into a composition of multiple vectors to express T9αH nucleic acid molecules and TOT nucleic acid molecules in two vectors, respectively.

In an embodiment of the disclosure, when the nucleic acid molecule composition includes a nucleic acid molecule encoding two bioenzymes: T9αH and TOT, it further includes a nucleic acid molecule encoding at least one of: TS, T5αH, T13αH, T2αH, T7βH, TAT and TBT, it can be prepared into a vector to express all nucleic acid molecules in one vector; or it can also be prepared into a composition of multiple vectors to express all nucleic acid molecules in multiple vectors, for example, expressing one or more nucleic acid molecules in one vector, as long as co-expression of all nucleic acid molecules can be achieved.

In an embodiment of the disclosure, a vector or a composition thereof, further includes a side chain esterification-related nucleic acid molecule encoding at least one of: phenylalanyl-CoA ligase (PCL), phenylalanine aminomutase (PAM), baccatin III 3-amino-3-phenylpropanoyl transferase (BAPT), cytochrome P450 hydroxylase (T2'αH), and 3'-N-debenzoyl-2'-deoxytaxol-N-benzoyl transferase (DBTNPT).

In an embodiment of the disclosure, a vector or a composition thereof further includes a nucleic acid molecule encoding geranylgeranyl pyrophosphate synthase (GGPPS).

In an embodiment of the disclosure, a vector further includes a transcriptional regulatory factor. For example, the transcriptional regulatory factor includes TcWRKY1, TcWRKY2, TcWRKY6, TcWRKY8, TcWRKY47, and TcMYC2a, etc.

In another embodiment of the disclosure, the biological material is a host cell or a composition thereof.

Optionally, the host cell includes at least one of: a microbial cell, a plant cell, an animal cell, and an algae cell.

In an embodiment of the disclosure, the host cell is a microbial cell.

In an embodiment of the disclosure, the microorganisms include bacteria and/or fungi.

In the embodiments of the disclosure, the host cell may be a type of host cell in which all nucleic acid molecules are expressed; or it may be a composition of multiple host cells in which all nucleic acid molecules are co-expressed through co-culture, multi-stage culture, and other methods.

In an embodiment of the disclosure, the host cell composition (a composition of multiple host cells) includes but is not limited to: a combination between bacteria, between fungi, and between bacteria and fungi, for example, a combination of E. coli and yeast. A composition consisting of different host cells can achieve the purpose of the disclosure through co-culture, multi-stage culture and the like.

In an embodiment of the disclosure, the bacteria include but are not limited to: *Escherichia* cells, *Lactobacillus* cells, *Lactococcus* cells, *Corynebacterium* bacteria, *Acetobacter* bacteria, *Acinetobacter* bacteria, *Pseudomonas* cells, *Streptomyces* cells, *Bacillus* cells, *Staphylococcus* cells, *Agrobacterium* cells, and endophytes of *Taxus.* For example, the bacteria include *Escherichia coli, Bacillus subtilis, Agrobacterium tumefaciens, Agrobacterium rhizogenes, Lactococcus lactis, Bacillus cereus, Pseudomonas fluorescens,* etc. In some embodiments, bacterial cells are, for example, *Escherichia* spp., *Streptomyces* spp., *Zymonas* spp., *Acetobacter* spp., *Citrobacter* spp., *Synechocystis* spp., *Rhizobium* spp., *Clostridium* spp., *Corynebacterium* spp., *Streptococcus* spp., *Xanthomonas* spp., *Lactobacillus* spp., *Lactococcus* spp., *Bacillus* spp., *Alcaligenes* spp., *Pseudomonas* spp., *Aeromonas* spp., *Azotobacter* spp., *Comamonas* spp., *Mycobacterium* spp., *Rhodococcus* spp., *Gluconobacter* spp., *Ralstonia* spp., *Acidithiobacillus* spp., *Microlunatus* spp., *Geobacter* spp., *Geobacillus* spp., *Arthrobacter* spp., *Flavobacterium* spp., *Serratia* spp., *Saccharopolyspora* spp., *Thermus* spp., *Stenotrophomonas* spp., *Chromobacterium* spp., *Sinorhizobium* spp., *Saccharopolyspora* spp., *Agrobacterium* spp., or *Pantoea* spp.. The bacterial cells may be Gram-negative cells, such as *E. coli* cells, or Gram-positive cells, such as *Bacillus* species.

In an embodiment of the disclosure, the fungus includes but is not limited to: yeast, filamentous fungi or mushrooms.

In a particular embodiment of the disclosure, yeast includes but is not limited to: *Saccharomyces cerevisiae, Schizosaccharomyces pombe, Yarrowia lipolytica, Candida utilis, Pichia pastoris, Kluyveromyces lactis, Hansenula polymorpha, Candida boidinii, Adenysera adenilyticus, Phaffia rhodozyma,* and *Candida albicans.* The yeast cells are, for example, *Saccharomyces* spp., *Schizosaccharomyces* spp., *Pichia* spp., *Paffia* spp., *Kluyveromyces* spp., *Candida* spp., *Talaromyces* spp., *Brettanomyces* spp., *Pachysolen* spp., *Debaryomyces* spp., *Yarrowia* spp. and industrial polyploid yeast strains. Preferably, the yeast strain is a *S*. *cerevisiae* strain or a *Yarrowia* strain.

In a particular embodiment of the disclosure, the filamentous fungi include but are not limited to: *Monascus, Aspergillus oryzae, Aspergillus niger, Aspergillus flavus,* and *Penicillium.* The fungi are, for example, *Aspergillus* spp., *Penicillium* spp., *Fusarium* spp., *Rhizopus* spp., *Acremonium* spp., *Neurospora* spp., *Sordaria* spp., *Magnaporthe* spp., *Allomyces* spp., *Ustilago* spp., *Botrytis* spp. and *Trichoderma* spp..

In an embodiment of the disclosure, the host cell is a plant cell.

In a particular embodiment of the disclosure, plant cells include but are not limited to: tobacco cells, *Pseudotaxus chienii* cells, *Artemisia annua* cells, *Arabidopsis* cells, *Physcomitrella patens* cells, *Marchantia polyphylla* cells, tomato cells, ginseng cells, cotton cells, sugarcane cells, potato cells, corn cells, wheat cells, rice cells, radish cells, lettuce cells, etc., and the cells include protoplasts, suspension cells, etc.

In an embodiment of the disclosure, the host cell is an animal cell.

In an embodiment of the disclosure, the animal cell includes insect cell, mammalian cell, nematode cell, and fish cell.

In a particular embodiment of the disclosure, insect cell includes but is not limited to: S2 *Drosophila* cells or Sf9 cell.

In a particular embodiment of the disclosure, mammalian cell includes but is not limited to: fibroblast, lymphocyte, epithelial cell, and myeloblast. For example, mammalian cell includes but is not limited to: HEK293 cell, CHO cell, COS cell, BHK cell, HeLa cell, Chinese hamster ovary cell, Vero cell, SP2/0 cell, NS/0 myeloma cell, hamster kidney cell, human B cell, human T cell Jurkat, neuronal cell, CV-I/EBNA cell, L cell, 3T3 cell, HEPG2 cell, and MDCK cell.

In a particular embodiment of the disclosure, fish cell includes but is not limited to zebrafish cell.

In an embodiment of the disclosure, the plant cell and/or animal cell include non-biologically transformed recombinant plant cell and/or recombinant animal cell.

Optionally, a plant cell or animal cell can express bioenzymes and produce target products through cell culture.

In an embodiment of the disclosure, the host cell is an algae cell.

In an embodiment of the disclosure, the algae cell includes but is not limited to at least one of: blue-green algae (cyanobacteria), green algae, *Synechococcus, Synechococcus elongatus, Synechocystis, Anabaena, Chlamydomonas,* and *Chlamydomonas reinhardtii.*

The disclosure also provides a method for producing a host cell, where the method includes transforming the host cell with at least one of the above nucleic acid molecules, expression cassettes or vectors in biological materials.

The disclosure further provides a method for producing a plants or plant cell, the meothd includes transforming the plant or plant cell with at least one of the above nucleic acid molecules, expression cassettes or vectors in biological materials.

The disclosure provides a method for producing the above bioenzyme composition, where a host cell is transformed by using the nucleic acid molecule composition of the disclosure, or an expression cassette or a composition thereof, a vector or a composition thereof, in the biological material, so that the host cell produces the bioenzyme composition.

In an embodiment of the disclosure, the host cell includes: microbial cell, plant cell, animal cell and/or algae cell.

In the above method, the method for transforming a host cell by using the nucleic acid molecule, expression cassette or vector includes but is not limited to: agrobacterium-mediated transformation, gene gun transformation, electroporation, polyethylene glycol (PEG) transformation, lipid transfection, heat shock, calcium phosphate precipitation, virus mediation, microinjection, and genetic engineering editing technology. The host cell includes microbial cell, plant cell, animal cell and/or algal cell.

In a particular embodiment of the disclosure, the method for producing a bioenzyme composition is, for example: (1) constructing a vector including the nucleic acid molecule composition of the disclosure; (2) transforming a host cell by using the obtained vector (the host cell includes microbial cell, plant cell, animal cell, algae cell, etc.); (3) culturing the obtained host cell to co-express the genes to produce Baccatin III.

In a particular embodiment of the disclosure, in the method for producing a bioenzyme composition, when nucleic acid molecules are expressed in multiple vectors, and the vectors include a vector for expressing at least two nucleic acid molecules, and a vector including multiple nucleic acid molecules can be constructed by using multivariate modular metabolic engineering (MMME).

In some embodiments, one or more nucleic acid molecules related to the disclosure are recombinantly expressed in bacterial cells. The bacterial cells of the disclosure can be cultured in culture medium of any type (rich or minimal) and of any composition. A person skilled in the art will appreciate that routine optimization will allow for the use of a variety of types of culture media. The selected culture medium may be supplemented with a variety of additional components. Some non-limiting examples of supplemental components include: glucose, antibiotics, IPTG for gene induction, ATCC trace mineral supplement, and glycolic acid. Likewise, other aspects of the culture medium and growth conditions for the cells of the disclosure can be optimized by routine experimentation. For example, pH and temperature are non-limiting examples of factors that can be optimized. In some embodiments, factors such as selection of culture medium, supplementation of culture medium, and temperature may affect the production level of taxane products such as baccatin III and paclitaxel. In some embodiments, the concentrations and amounts of supplemental components may be optimized. In some embodiments, the frequency of supplementing the culture medium with one or more supplemental components, and the culturing time of the medium prior to harvesting the terpenoid (such as taxadiene), are optimized.

Liquid cultures used to grow cells related to the disclosure may be stored in any culture container known and used in the art. In some embodiments, large-scale production in aerated reaction vessels (e.g., stirred tank reactors) can be used to generate large quantities of taxane product, which can be recovered from the cell culture. In some embodiments, the taxane product is recovered from the gas phase of the cell culture, for example, by adding an organic layer such as dodecane to the cell culture and recovering the taxane product from the organic layer.

In some embodiments, taxane product is recovered, for example, by adding an organic layer such as ethyl acetate to the cell culture and recovering the product from the organic layer.

The disclosure provides use of any one of the bioenzyme composition, nucleic acid molecule composition, and biomaterial of the disclosure in the synthesis of baccatin III and/or an intermediate thereof.

In an embodiment of the disclosure, the intermediate of baccatin III includes a compound represented by the following Formula II and/or Formula III: 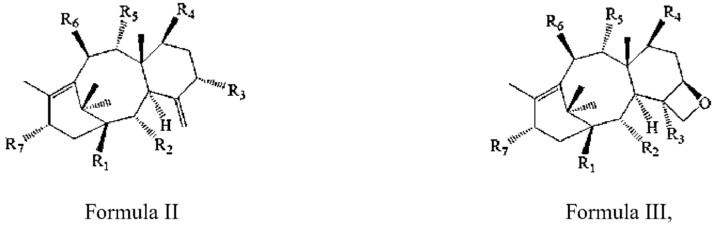 where R₁, R₃ and R₄ are independently selected from H, -OH and -OAc (acetoxyl, -OOCCH₃); R₂ and R₆ are selected from H, -OH, -OAc and -OBz (benzoyl, C₆H₅-CO-CH₂-); and R₅ and R₇ are selected from H, -OH, =O and -OAc.

In an embodiment of the disclosure, baccatin III further includes baccatin III derivatives.

The disclosure provides a method for synthesizing baccatin III and/or an intermediate thereof, where under the action of the bioenzyme composition according to the disclosure and/or the bioenzyme composition produced by the above method, the precursor of baccatin III and/or an intermediate thereof is used as a substrate to catalyze hydroxylation at C9 position of taxane ring skeleton and formation of C4-C20 oxetane of the taxane ring skeleton, thereby synthesizing baccatin III and/or an intermediate thereof.

In an embodiment of the disclosure, a host cell is transformes by using the nucleic acid molecule composition or any one of the biological materials (e1)-(e2) according to the disclosure, so that the host cell produce a bioenzyme composition; where the host cell includes at least one of: microbial cell, plant cell, animal cell and algae cell; under the action of the produced bioenzyme composition, the precursor of baccatin III and/or an intermediate thereof is used as a substrate to catalyze hydroxylation of C9 position of taxane ring skeleton and formation of C4-C20 oxetane of the taxane ring skeleton, thereby synthesizing baccatin III and/or an intermediate thereof.

In an embodiment of the disclosure, the method for synthesizing baccatin III and/or an intermediate thereof further includes any one of the following reactions (f1) to (f3):
(f1) hydroxylation of at least one carbon position of C1, C2, C5*,* C7, C10 and C13 of the taxane ring backbone;
(f2) acylation of at least one hydroxyl group of C2, C5 and C10; and
(f3) ketonization of C9 hydroxyl.

In an embodiment of the disclosure, the host cell is a microbial cell, and the method for synthesizing baccatin III and/or an intermediate thereof further includes a step of fermentation culture.

In an embodiment of the disclosure, the host cell is a plant cell or an animal cell, and the method for synthesizing baccatin III and/or an intermediate thereof further includes a step of cell culture.

Optionally, the culture method includes: immobilized cell culture, two-stage culture, two-phase culture, addition of inducers (such as salicylic acid, silver nitrate, methyl jasmonate, arachidonic acid, ammonium citrate, etc.), addition of bypass inhibitors (such as chlormequat), addition of substrates (such as GGPP, taxadiene and/or other baccatin III intermediates), etc.

In an embodiment of the disclosure, the host cell is a plant cell, and optionally the method for synthesizing Baccatin III and/or an intermediate thereof further includes the steps of planting plants, harvesting plants, and extracting products.

In an embodiment of the disclosure, the method for synthesizing baccatin III includes under the action of at least six p450 enzymes (i.e., T2αH, T5αH, T7βH, T9αH, T13αH and TOT) and two cytoplasmic acyltransferases (TAT and TBT), synthesizing baccatin III by using taxadiene as a substrate.

In a particular embodiment of the disclosure, the substrate taxadiene is synthesized under the action of TS enzyme.

The disclosure provides use of any one of the bioenzyme composition, nucleic acid molecule composition, and biomaterial according to the disclosure in the synthesis of a taxane compound and/or an intermediate thereof.

The disclosure provides a method for synthesizing a taxane compound and/or an intermediate thereof, the method includes the following steps:
synthesizing baccatin III by the above method;
using baccatin III as a substrate, and further catalyzing to synthesize the taxane compound and/or an intermediate thereof.

The above method for synthesizing Baccatin III, a taxane compound and/or an intermediate thereof further includes an optimization step. The optimization step includes at least one of the following optimization methods:

Optimization of Gene Expression: for example, codon optimization, optimization of transcriptional regulatory factor, promoter optimization, and optimization of fusion protein, etc. Codon optimization (including identification of optimal codons for various organisms, and methods for achieving codon optimization) is well known to those of ordinary skill in the art, and can be accomplished using standard methods. The transcriptional regulator ORCA3 gene is a MeJA-induced transcriptional regulator that regulates plant basal and secondary metabolism. Optimization of gene expression can also be achieved by selecting appropriate promoters and ribosome binding sites. In some embodiments, optimization of gene expression may include selecting for a high copy number plasmid, or a low or medium copy number plasmid. Steps in transcription termination can also be targeted to regulate gene expression by introducing or eliminating structures such as stem-loops.

Optimization of Metabolic Engineering: for example, some methods for increasing the production of secondary metabolites through metabolic engineering, such as overcoming rate-limiting steps, reducing metabolic flux to competing pathways, reducing catabolism, and overexpressing regulatory genes.

Optimization of Regulatory Factors: for example, adding MeJA and an analog thereof, salicylic acid, arachidonic acid, coronatine, etc. to regulate biosynthesis.

Optimization of Culture Methods: for example, improving culture medium, adjusting environmental factors such as culture temperature and time, optimizing culture process, combined culture, etc.; for example, Zhou K et al. gave a culture example of co-culture of *E. coli* and yeast (Zhou, K., Qiao, K., Edgar, S., and Stephanopoulos, G. (2015). Distributing a metabolic pathway among a microbial consortium enhances production of natural products. NAT BIOTECHNOL 33, 377-383).

In an embodiment of the disclosure, the taxane compounds include but are not limited to at least one of: paclitaxel and a derivative thereof, taxusin and a derivative thereof, docetaxel and a derivative thereof, and cabazitaxel and a derivative thereof.

In an embodiment of the disclosure, intermediates of taxane compound include but are not limited to: β-phenylalanoyl baccatin III, and 3'-N-debenzoyltaxol.

The disclosure provides a plant or plant parts thereof, where the plant is one of the following plants:
(g1) a plant formed by growing the above plant cell;
(g2) a plant obtained by the above method for producing a bioenzyme composition in a plant;
(g3) a progeny formed by self-pollination of any plant in (g1) to (g2), and a plant grown from the progeny;
(g4) a progeny formed by hybridization of any plant in (g1) to (g2) with other variety, and a plants grown from the progeny; and
the above plant parts are root, stem, leaf, flower, fruit, pollen or seed.

The disclosure provides a plant or a plant part thereof, where the plant includes a transgenic plant including the above nucleic acid molecule composition.

The disclosure further provides a method for preparing a plant or a plant part thereof, where method includes the steps of tissue culture and/or induction culture.

The disclosure further provides a method for manufacturing a commercial product, the method includes: obtaining the plant or plant part according to the ninth or tenth aspect thereof, and manufacturing the commercial product from the plant or plant part thereof; where the commercial product is a crude extract, a raw material medicine, and/or a pharmaceutical formulation including at least one selected from the group consisting of: baccatin III and a derivative thereof, paclitaxel and a derivative thereof, taxusin and a derivative thereof, docetaxel and a derivative thereof, and cabazitaxel and a derivative thereof.

The disclosure also provides a product generated by any one of the above methods, where the product includes at least one of the following ingredients: bacatine III and a derivative thereof, paclitaxel and a derivative thereof, taxusin and a derivative thereof, docetaxel and a derivative thereof, and cabazitaxel and a derivative thereof.

Compared with the prior art, the disclosure has the following beneficial effects:
The disclosure successfully identified two essential genes for the synthesis of baccatin III: gene of taxane C4-C20 oxetanase (TOT), and gene of taxane 9α-hydroxylase (T9αH). Particularly, TOT has the functions of two enzymes previously speculated by researchers, i.e., C4-C20 epoxidase (C4β, C20-epoxidase, EPOX) and oxomutase (OXM), thereby catalyzing the oxidation of carbon-carbon double bonds of the taxane molecule into oxetane and oxirane.

Furthermore, the disclosure co-expressed the two new genes TOT and T9αH and other known genes involved in the biosynthesis of baccatin III (such as TS, T5αH, T13αH, T2αH, T7βH, TAT and TBT), and surprisingly found that these 9 genes are able to successfully reconstruct the biosynthetic pathway of baccatin III from GGPP to baccatin III in tobacco. The results show that these nine enzymes are the core components of the biosynthesis from GGPP to baccatin III, including taxadiene synthase (TS), two acyl transferases, and six CYP450 enzymes. The C9 oxidase (T9αO) and C1 hydroxylase (T1βH) speculated by previous researchers may be replaced by other enzymes in the gene combination of the disclosure. The research results of this disclosure revealed an unprecedented reaction mechanism of oxidative rearrangement, identified the core components of the biosynthetic pathway of Baccatin III, thereby constructing an artificial synthesis route of Baccatin III.

Furthermore, in the prior art, the essential genes T10βH and 10-deacetylbaccatin III-10-β-O-acetyl transferase (DBAT) have been considered to be necessary for the biosynthesis of baccatin III. In the method for the biosynthesis of baccatin III according to the disclosure, baccatin III can still be synthesized in the absence of these two enzymes. The research of the disclosure shows that T10βH and DBAT are not essential, and the functions may be performed by other enzymes in the gene combination of the disclosure.

Furthermore, by successfully constructing the complete biosynthetic pathway of baccatin III in tobacco, a new enzymatic mechanism in the biosynthesis of paclitaxel was revealed, thereby filling the key gap in its complete biosynthetic pathway, and paving the way for efficiently developing a green low-carbon production pathway of taxane products (such as paclitaxel) through synthetic biology.

### BRIEF DESCRIPTION OF THE DRAWINGS

Fig. 1 is a schematic diagram of the biosynthetic pathway of paclitaxel.
Fig. 2 shows the synthesis of Baccatin III in tobacco by using different gene combinations in Example 3;
   particularly, Baccatin III corresponds to the chromatographic elution curve of the baccatin III control sample; 7 represents product peak 7;
   Group 1 corresponds to the chromatographic elution curve of the product synthesized by the combination of 9 genes (T9αH, TOT, TS, TAT, T5αH, T13αH, T2αH, T7βH, and TBT);
   Groups 2-10 correspond to the chromatographic elution curves of the products synthesized by the combinations formed by respectively lacking one gene in the above 9 genes.

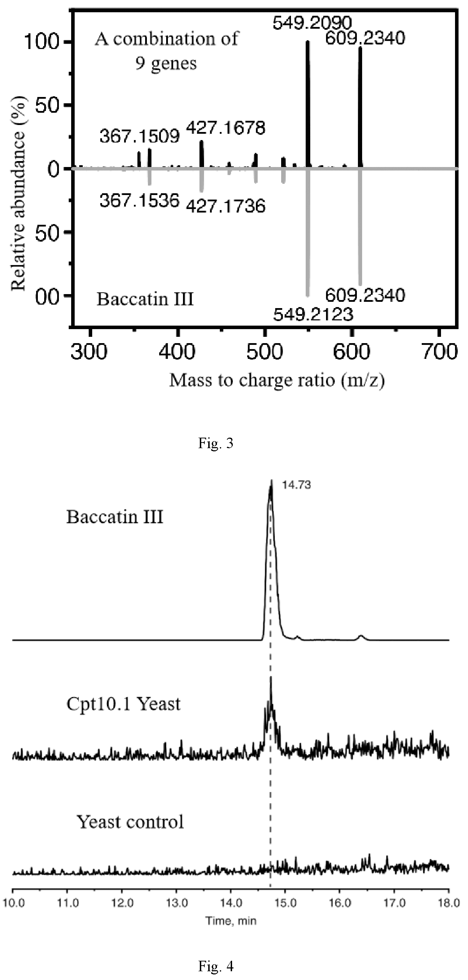

Fig. 3 shows the results of tandem mass spectrometry (MS/MS) analysis of the products synthesized by Group 1.
Fig. 4 shows the chromatographic elution curves of baccatin III produced in yeast in Example 4, as well as the comparison graphs with the chromatographic elution curves of baccatin III control sample and yeast control.

The meanings of the terms in the drawings are as follows:
baccatin III: baccatin III;
Group: Group;
EIC: Extract Ion Chromatogram;
Retention time: Retention time;
Relative abundance: Relative abundance;
GGPPS: geranylgeranyl pyrophosphate synthase;
TS: taxadiene synthase;
PAM: phenylalanine isomerase;
IPP: isopentenyl pyrophosphate;
DMAPP: dimethylallyl pyrophosphate;
GGPP: geranylgeranyl pyrophosphate.
Time, min: Time, min.

### DETAILED DESCRIPTION OF THE EMBODIMENTS

The disclosure discloses a method for biological total synthesis of paclitaxel key precursor substance baccatin III, a biological material and use thereof. Those skilled in the art can refer to the content of this disclosure and appropriately improve the process parameters to achieve it. It should be particularly noted that, all similar substitutions and modifications are obvious to those skilled in the art, and they are deemed to be included in the disclosure. The method and use according to the disclosure have been described through preferred embodiments. Relevant personnel can obviously modify or appropriately modify and combine the method and use described herein without departing from the content, spirit and scope of the disclosure to implement and apply the technology of the disclosure.

### Terminology explanation:

In the disclosure, the term "bioenzyme" or "enzyme" refers to a protein (or "polypeptide" or "peptide composition") with biological activity, including both naturally occurring proteins and variants and modified forms thereof. As used herein, the terms "protein" and "polypeptide" are used interchangeably, and thus the term polypeptide can be used to refer to a full-length polypeptide as well as a fragment of a full-length polypeptide. The term "fragment" refers to a portion of a polypeptide sequence. A "fragment" or "biologically active portion" includes a polypeptide including a sufficient number of contiguous amino acid residues to retain biological activity, such as a polypeptide in which the N-terminal amino acid has been truncated.

"Variant" means a substantially similar sequence. As will be readily appreciated by those skilled in the art, naturally occurring proteins may have some differences between different species of the same genus or between different samples of the same species, which may be deletion and/or addition of one or more amino acids at one or more internal sites and/or substitution of one or more amino acids at one or more sites of the native polypeptide. However, this does not affect their ability to play the same or similar roles, and these proteins can be referred to as natural variants of the exemplary proteins. Modifications of proteins include, but are not limited to, appropriate amino acid substitutions/additions/deletions, truncation of N-terminal amino acid, codon optimization suitable for host cell preferences, tag addition and fusion, etc., which do not affect the biological activity of the target protein. These proteins may be referred to as artificial variants of the exemplary proteins. Guidance for appropriate amino acid substitutions that do not affect the biological activity of the target protein can refer to the model described in Dayhoff et al. (1978) Atlas of Protein Sequence and Structure (Natl.Biomed.Res.Found., Washington,D.C.), which is incorporated herein by reference. Conservative substitutions, such as replacing one amino acid with another having similar properties, may be optimal.

In the disclosure, the term "amino acid" refers to any amino acid (both standard and non-standard amino acids), including but not limited to: α-amino acid, β-amino acid, γ-amino acid and δ-amino acid. Examples of suitable amino acids include, but are not limited to, alanine, asparagine, aspartic acid, cysteine, glutamic acid, glutamine, glycine, proline, serine, tyrosine, arginine, histidine, isoleucine, leucine, lysine, methionine, phenylalanine, threonine, tryptophan, and valine.

In the disclosure, the term "taxadiene synthase" refers to an enzyme that catalyzes the cyclization of GGPP to produce taxadiene (taxa-4(5),11(12)-diene), and its exemplary amino acid sequences or nucleotide sequences are shown in Table 1. Those skilled in the art can also obtain other amino acid sequences or nucleotide sequences with the same function through databases or literature reports. This enzymatic reaction generates the unique skeleton structure of taxane compounds, so this enzyme is considered to be the key enzyme that determines whether the precursor complex can synthesize paclitaxel. In the disclosure, the term "TS" may refer to taxadiene synthase, or a taxadiene synthase gene, or a nucleotide molecule or sequence encoding taxadiene synthase, and the specific meaning may be determined in combination with the context. The content disclosed in Chinese patent disclosure CN202010647327.3 is incorporated herein by reference in its entirety.

In the disclosure, the term "taxane 5α-hydroxylase" refers to an enzyme that catalyzes the hydroxylation of the C5 position of the taxane backbone, and its exemplary amino acid sequences or nucleotide sequences are shown in Table 1. Those skilled in the art can also learn other amino acid sequences or nucleotide sequences with the same function through databases or literature reports. In the disclosure, the term "T5αH" may refer to taxane 5α-hydroxylase, or taxane 5α-hydroxylase gene, or a nucleotide molecule or sequence encoding taxane 5α-hydroxylase, and the specific meaning may be determined in combination with the context.

In the disclosure, the term "taxane 13α-hydroxylase" refers to an enzyme that catalyzes the hydroxylation of the C13 position of the taxane backbone, and its exemplary amino acid sequences or nucleotide sequences are shown in Table 1. Those skilled in the art can also learn other amino acid sequences or nucleotide sequences with the same function through databases or literature reports. In the disclosure, the term "T13αH" may refer to taxane 13α-hydroxylase, or taxane 13α-hydroxylase gene, or a nucleotide molecule or sequence encoding taxane 13α-hydroxylase, and the specific meaning may be determined in combination with the context.

In the disclosure, the term "taxane 2α-hydroxylase" refers to an enzyme that catalyzes the hydroxylation of the C2 position of the taxane skeleton, and its exemplary amino acid sequences or nucleotide sequences are shown in Table 1. Those skilled in the art can also obtain other amino acid sequences or nucleotide sequences with the same function through databases or literature reports. In the disclosure, the term "T2αH" may refer to taxane 2α-hydroxylase, or taxane 2α-hydroxylase gene, or a nucleotide molecule or sequence encoding taxane 2α-hydroxylase, and the specific meaning may be determined in combination with the context.

In the disclosure, the term "taxane 7β-hydroxylase" refers to an enzyme that catalyzes the hydroxylation of the C7 position of the taxane backbone, and its exemplary amino acid sequences or nucleotide sequences are shown in Table 1. Those skilled in the art can also obtain other amino acid sequences or nucleotide sequences with the same function through databases or literature reports. In the disclosure, the term "T7βH" may refer to taxane 7β-hydroxylase, or taxane 7β-hydroxylase gene, or a nucleotide molecule or sequence encoding taxane 7β-hydroxylase, and the specific meaning may be determined in combination with the context.

In the disclosure, the term "taxane 5α-acetyl transferase" refers to an enzyme that catalyzes the acetylation of the C5 oxidation product of taxane, and its exemplary amino acid sequences or nucleotide sequences are shown in Table 1. It may also have the function of catalyzing the acetylation of the C10 oxidation product of taxane. Those skilled in the art may also know other amino acid sequences or nucleotide sequences with the same function through databases or literature reports. In the disclosure, the term "TAT" may refer to taxane 5α-acetyl transferase, or taxane 5α-acetyl transferase gene, or a nucleotide molecule or sequence encoding taxane 5α-acetyl transferase, and the specific meaning may be determined in combination with the context.

In the disclosure, the term "taxane 2α-O-benzoyl transferase" refers to an enzyme that catalyzes the benzoylation of the C2 oxidation product of taxane, and its exemplary amino acid sequences or nucleotide sequences are shown in Table 1. Those skilled in the art can also obtain other amino acid sequences or nucleotide sequences with the same function through databases or literature reports. In the disclosure, the term "TBT" may refer to taxane 2α-O-benzoyl transferase, or taxane 2α-O-benzoyl transferase gene, or a nucleotide molecule or sequence encoding taxane 2α-O-benzoyl transferase, and the specific meaning may be determined in combination with the context.

In the disclosure, the term "taxane 9α-hydroxylase" refers to an enzyme that catalyzes the hydroxylation of the C9 position of the taxane skeleton. According to the experimental results of the disclosure, it can also have the function of catalyzing the carbonylation of the C9 position of the taxane ring skeleton. Its exemplary amino acid sequences or nucleotide sequences are shown in Table 1. In the disclosure, the term "T9αH" may refer to taxane 9α-hydroxylase, or taxane 9α-hydroxylase gene, or a nucleotide molecule or sequence encoding taxane 9α-hydroxylase, and the specific meaning may be determined in combination with the context. The content disclosed in Chinese patent disclosure CN202310961179.6 is incorporated herein by reference in its entirety.

In the disclosure, the term "taxane C4-C20 oxetanase" refers to an enzyme that catalyzes the oxidation of carbon-carbon double bonds of taxane molecule into oxetane and oxirane, such as catalyzing the generation of 1-dehydroxybaccatin IV and baccatin I from taxadiene-hexol-hexaacetate, and its exemplary amino acid sequences or nucleotide sequences are shown in Table 1. In the disclosure, the term "TOT" may refer to taxane C4-C20 oxetanase, or taxane C4-C20 oxetanase gene, or a nucleotide molecule or sequence encoding taxane C4-C20 oxetanase, and the specific meaning may be determined in combination with the context. The content disclosed in Chinese patent disclosure CN202310496624.6 is incorporated herein by reference in its entirety.

In some embodiments, the bioenzymes related to the disclosure can be isolated from a material including a given bioenzyme from any source. Any method of obtaining the bioenzymes related to the disclosure is compatible with the disclosure.

As used herein, the term "isolating" means removing from its natural environment or from other compound present when the compound is first formed. The term "isolated" includes a material separated from a natural source, as well as a material recovered following production by recombinant expression in a host cell (such as nucleic acids and proteins), or a chemically synthesized compound (such as a nucleic acid molecule, protein and peptide).

In the disclosure, the term "nucleic acid molecule" (or "nucleic acid" or "polynucleotide") may refer to a polymeric form of nucleotides, which may include sense and antisense strands of RNA, cDNA, genomic DNA, as well as synthetic forms and mixed polymers thereof. A nucleotide may refer to ribonucleotide, deoxyribonucleotide, or modified form of either type of nucleotide. As used herein, "nucleic acid molecule" is synonymous with "nucleic acid" and "polynucleotide." A nucleic acid molecule is usually at least 10 bases in length unless otherwise specified. The term can refer to an RNA or DNA molecule of indeterminate length. The term includes both single-stranded and double-stranded forms of DNA. Nucleic acid molecules can include one or both of naturally occurring and modified nucleotides linked together by naturally occurring and/or non-naturally occurring nucleotide linkages.

As will be readily appreciated by those skilled in the art, for nucleotide sequences, well-known molecular biology techniques, such as the polymerase chain reaction (PCR) and hybridization techniques outlined herein, can be used to identify naturally occurring variants, i.e., substantially similar sequences.

As will be readily appreciated by those skilled in the art, nucleic acid molecules may be chemically or biochemically modified, or may include non-natural or derived nucleotide bases. Such modifications include, for example, labeling, methylation, substitution of one or more naturally occurring nucleotides with an analog, internucleotide modifications (e.g., uncharged bonds: e.g., methylphosphonate, phosphotriester, phosphoramidite, carbamate, etc.; charged bonds: e.g., phosphorothioate, phosphorodithioate, etc.; pendant moieties: e.g., peptide; intercalator: e.g., acridine, psoralen, etc.; chelating agents; alkylating agents; and modified bonds: e.g., α-anomeric nucleic acids, etc.). The term "nucleic acid molecule" further includes any topological conformation, including single-stranded, double-stranded, partially double-stranded, triple-stranded, hairpin, circular, and padlocked conformations.

In the disclosure, the term "identity" refers to sequence similarity with natural nucleic acid sequences or amino acid sequences. Identity can be assessed by the naked eye or by computer software. Using computer software, the identity between two or more sequences can be expressed as a percentage (%), which can be used to evaluate the identity between related sequences.

Unless otherwise indicated, the sequence identity values provided herein refer to the values obtained using the full-length sequences of the disclosure by Jalview version 2.11.2.7 (Waterhouse, A.M., Procter, J.B., Martin, D.M.A, Clamp, M. and Barton, G.J. (2009) "Jalview Version 2 -- a multiple sequence alignment editor and analysis workbench" Bioinformatics 25(9) 1189-1191 doi:10.1093/bioinformatics/btp033), and using the default parameters in the multiple alignment software package MUSCLE version v3.8.31 ("MUSCLE: multiple sequence alignment with high accuracy and high throughput" Nucleic Acids Res. 32(5):1792 (2004)); or any equivalent program thereof).

Additional mathematical algorithms are known in the art and can be used to align two sequences. See, for example, the algorithms in Karlin and Altschul (1990) Proc. Natl.Acad.Sci.USA 87:2264, such as a modification in Altschul(1993)Proc.Natl.Acad.Sci.USA 90:5873-5877. This algorithm was introduced in the BLAST program of Altschul et al. (1990) J.Mol. Biol. 215:403. BLAST nucleotide searches can be performed with the BLASTN program (nucleotide query for nucleotide sequence searches) to obtain nucleotide sequences homologous to the nucleic acid molecules of the disclosure, or with the BLASTX program (translated nucleotide query for protein sequence searches) to obtain protein sequences homologous to the nucleic acid molecules of the disclosure. BLAST protein searches can be performed with the BLASTP program (protein query for protein sequence searches) to obtain amino acid sequences homologous to protein molecules of the disclosure, or with the TBLASTN program (protein query for translated nucleotide sequence searches) to obtain nucleotide sequences homologous to protein molecules of the disclosure. To obtain gapped alignments for comparison purposes, Gapped BLAST (in BLAST 2.0) can be used as described in Altschul et al. (1997) Nucleic Acids Res. 25:3389. Alternatively, PSI-Blast can be used to perform an iterated search that detects distant relationships between molecules. See Altschul et al. (1997) supra. When utilizing BLAST, Gapped BLAST, and PSI-Blast programs, the default parameters of the respective programs (e.g., BLASTX and BLASTN) can be used. Alignment can also be performed manually by inspection.

In the disclosure, the term "homology" is sometimes used to refer to the level of similarity (i.e., sequence similarity or identity) between two or more nucleic acid sequences or amino acid sequences expressed as a percentage of positional identity. Homology also refers to the concept of evolutionary relatedness, often demonstrated by similar functional properties between different nucleic acids or proteins that share similar sequences.

In some embodiments, homologous sequences related to the disclosure can be obtained by comparing exemplary sequences in genome or transcriptome data of samples of species with close evolutionary relationships. For example, between different species of the same genus, or between different plants of the same species, by comparing the exemplary sequence in the sample genome or transcriptome data to obtain the homologous sequence of said sequence, those skilled in the art can expect that it has the same or similar functions.

In some embodiments, nucleic acid molecules related to the disclosure can be cloned from DNA including a given nucleic acid molecule from any source, for example, by PCR amplification and/or restriction enzyme digestion. In some embodiments, nucleic acid molecules related to the disclosure are synthetic. Any method for obtaining a nucleic acid molecule related to the disclosure is compatible with the disclosure.

As used herein, the term "synthetic" refers to polynucleotide (i.e., DNA or RNA) molecules produced by chemical synthesis as an in vitro process. For example, synthetic DNA can be produced in a reaction process within an Eppendorf^{™} tube, such that synthetic DNA is enzymatically produced from a natural DNA or RNA strand. Other laboratory methods may be utilized to synthesize polynucleotide sequences. Oligonucleotides can be chemically synthesized on an oligonucleotide synthesizer by solid phase synthesis using phosphoramidites. Synthetic oligonucleotides can anneal to each other as a complex, thereby producing a "synthetic" polynucleotide. Other methods for chemically synthesizing polynucleotides are known in the art, and can be readily adapted for use with the present disclosure.

In the disclosure, the term "gene" refers to a nucleic acid fragment expressing a specific protein. A "gene" includes DNA regions that encode a gene product, as well as all DNA regions that regulate the production of a gene product, whether or not such regulatory sequences are adjacent to the coding and/or transcribed sequences. Thus, genes include, but are not necessarily limited to: promoter sequences, terminators, translation regulatory sequences, such as ribosome binding sites and internal ribosome entry sites, enhancers, silencers, insulators, boundary elements, replication origins, matrix attachment sites, introns, and locus control regions.

In the disclosure, the term "gene product" refers to any product generated by a gene. For example, a gene product can be a direct transcription product of the gene (such as mRNA, tRNA, rRNA, antisense RNA, interfering RNA, ribozyme, structural RNA, or any other type of RNA), or it can be a protein produced by translation of mRNA.

In the disclosure, the term "expression cassette" refers to a DNA fragment that can be inserted into a nucleic acid or polynucleotide at a specific restriction site or by homologous recombination. As used herein, a DNA fragment includes a polynucleotide encoding a polypeptide of interest, and an expression cassette and restriction sites are designed to ensure insertion of the expression cassette into the proper reading frame for transcription and translation. In one embodiment, an expression cassette may include a polynucleotide encoding a polypeptide of interest and, in addition to the polynucleotide, have elements that facilitate transformation of a particular host cell. In one embodiment, the expression cassette may further include elements that allow for enhanced expression of the polynucleotide encoding the polypeptide of interest in the host cell. These elements may include, but are not limited to, promoters, minimal promoters, enhancers, response elements, terminator sequences, polyadenylation sequences, and the like.

The expression cassette may also include a selectable marker gene for selecting transformed cells. Selectable marker genes are used to select transformed cells or tissues. Marker genes include genes encoding antibiotic resistance, such as genes encoding neomycin phosphotransferase II (NEO) and hygromycin phosphotransferase (HPT), and genes conferring resistance to herbicidal compounds (such as glufosinate, bromoxynil, imidazolinone, and 2,4-dichlorophenoxyacetate (2,4-d)). Additional selectable markers include phenotypic markers, for example, β-galactosidase and fluorescent proteins, such as green fluorescent protein (GFP), cyan fluorescent protein (CYP), and yellow fluorescent protein.

In the disclosure, the term "vector" is used interchangeably with "construct", "cloning vector" and "expression vector", and means a vector that can introduce DNA or RNA sequences (such as foreign genes) into host cells to transform the host and promote the expression (such as transcription and translation) of the introduced sequences. "Non-viral vector" refers to any vector that does not include a virus or retrovirus. In some embodiments, a "vector" is a DNA sequence including at least one DNA replication origin and at least one selectable marker gene. Examples include, but are not limited to, plasmids, cosmids, bacteriophages, bacterial artificial chromosomes (BACs), or viruses that carry foreign DNA into cells. The vector may further include one or more genes, antisense molecules and/or selectable marker genes and other genetic elements known in the art. The vector may transduce, transform or infect cells, thereby causing the cells to express the nucleic acid molecules and/or proteins encoded by the vector. The term "plasmid" refers to a circular chain of nucleic acid capable of autosomal replication in a prokaryotic or eukaryotic host cell. The term includes nucleic acids, which may be DNA or RNA, and which may be single-stranded or double-stranded. A plasmid as defined may further include a sequence corresponding to a bacterial origin of replication.

In some embodiments, a "cloning vector" is capable of autonomous replication or integration in the host cell genome, and is further characterized by one or more restriction endonuclease sites at which the vector can be cut in a defined manner and a desired DNA sequence can be ligated into the vector, such that the new recombinant plasmid retains its ability to replicate in the host cell. In the case of a plasmid, replication of the desired sequence may occur multiple times as the plasmid increases in copy number in a host cell (such as a bacterial host), or only once per host before the host reproduces by mitosis. In the case of bacteriophages, replication can occur actively during the lytic phase or passively during the lysogenic phase.

In some embodiments, an "expression vector" can be used to insert a desired DNA sequence into it by restriction enzyme digestion and ligation, so that it is operably linked to a regulatory sequence and can be expressed as an RNA transcript. The vector may also include one or more marker sequences suitable for identifying cells that have been transformed or transfected with the vector. Markers include, for example, genes encoding proteins that increase or decrease resistance or sensitivity to antibiotics or other compounds, genes encoding enzymes whose activity can be detected by standard methods known in the art (e.g., β-galactosidase, luciferase, or alkaline phosphatase), and genes that have a visible effect on the phenotype of transformed or transfected cells, hosts, colonies or plaques (e.g., green fluorescent protein). Preferred vectors are those capable of autonomous replication and expression of the structural gene products present in the DNA fragments to which they are operatively linked.

In the disclosure, the term "expression" refers to the biosynthesis of a gene product, including the transcription and/or translation of the gene product. "Expressing" or "producing" a protein or polypeptide from a DNA molecule refers to transcribing and translating the coding sequence to produce the protein or polypeptide, while "expressing" or "producing" a protein or polypeptide from an RNA molecule refers to translating the RNA coding sequence to produce the protein or polypeptide.

Gene expression can be influenced by external signals, for example, exposure of a cell, tissue or organism to a substance that increases or decreases gene expression. Gene expression can also be regulated anywhere along the way from DNA to RNA to protein. Regulation of gene expression can be achieved through control of transcription, translation, RNA transport and processing, degradation of intermediate molecules (such as mRNA), or through activation, inactivation, compartmentalization or degradation of specific protein molecules after their production, or through a combination of these. The precise nature of the regulatory sequences required for gene expression may vary between species or cell types, but will generally include, as necessary, 5' non-transcribed and 5' non-translated sequences involved in the initiation of transcription and translation, respectively, such as a TATA box, capping sequence, CAAT sequence, and the like. In particular, such 5' non-transcribed regulatory sequences will include a promoter region, which includes a promoter sequence that controls the transcriptional control of an operably linked gene. Regulatory sequences may further include enhancer sequences or desired upstream activator sequences. The vectors of the disclosure may optionally include a 5' leader or signal sequence. The selection and design of appropriate vectors is within the ability and judgment of one of ordinary skill in the art.

In some embodiments, when a nucleic acid molecule encoding any enzyme of the disclosure is expressed in a cell, a variety of transcription control sequences (e.g., promoter/enhancer sequences) can be used to direct its expression. The promoter may be a natural promoter, i.e., the promoter of a gene in its endogenous environment, which provides for normal regulation of gene expression. In some embodiments, a promoter may be constitutive, i.e., the promoter continues to transcribe its associated gene without regulation. Multiple conditional promoters, such as promoters that are controlled by the presence or absence of a molecule, may also be used. Chemically regulated promoters can be used to regulate the expression of genes in a host by applying exogenous chemical regulators. Depending on the purpose, the promoter can be a chemical-inducible promoter, in which disclosure of the chemical induces gene expression, or a chemical-repressible promoter, in which disclosure of the chemical represses gene expression. Chemical-inducible promoters are known in the art, and include, but are not limited to: the maize In2-2 promoter (which is activated by benzenesulfonamide herbicide safeners), the maize GST promoter (which is activated by hydrophobic electrophilic compounds used as pre-emergence herbicides), and the tobacco PR-1a promoter (which is activated by salicylic acid). Other chemically regulated promoters of interest include glucocorticoid-inducible promoters among the steroid-responsive promoters, as well as tetracycline-inducible and tetracycline-repressible promoters.

Expression vectors including all necessary expression elements are commercially available, and familiar to those skilled in the art. See, e.g., Sambrook et al., Molecular Cloning: A Laboratory Manual, 2nd ed., Cold Spring Harbor Laboratory Press, 1989. Cells are genetically engineered by introducing exogenous DNA (RNA) into the cells. The exogenous DNA (RNA) is placed under the effective control of transcription elements to allow the exogenous DNA to be expressed in the host cell.

In the disclosure, the term "transformation" includes all techniques by which a nucleic acid molecule can be introduced into such a cell. Examples include, but are not limited to: transfection with viral vectors; transformation with plasmid vectors; electroporation; fat infection; microinjection; agrobacterium-mediated transfer; direct DNA uptake; WHISKERS^{™} mediated transformation; and microprojectile bombardment. These techniques can be used for both stable and transient transformation of host cells. "Stable transformation" refers to the introduction of a nucleic acid fragment into the genome of a host organism, resulting in genetic stability. Once stably transformed, the nucleic acid fragment is stably integrated into the genome of the host organism and any subsequent generations. Host organisms including the transformed nucleic acid fragments are referred to as "transgenic" organisms. "Transient transformation" refers to the introduction of a nucleic acid fragment into the nucleus or DNA-including organelle of a host organism, resulting in gene expression without genetically stable inheritance.

In some embodiments, to transform hosts and host cells, the nucleotide sequence of the disclosure can be inserted into any vector known in the art suitable for expressing nucleotide sequences in hosts or host cells by using standard techniques. The choice of vector depends upon the preferred transformation technique and the target host species to be transformed. The transformation method depends on the host cell to be transformed, the stability of the vector used, the expression level of the gene product and other parameters.

In the disclosure, the term "plant" includes seeds, plant cells, plant protoplasts, plant cell tissue cultures from which plants can be regenerated, plant callus, plant pieces, and plant cells that are intact in plants or plant parts such as embryos, pollen, ovules, seeds, tubers, propagules, leaves, flowers, branches, fruits, roots, root tips, anthers, etc. Progeny, variants and mutants of the regenerated plants are further included within the scope of the disclosure, provided that such parts contain the introduced polynucleotides. As used herein, unless expressly stated otherwise or clear from the context of use, "progeny" and "progeny plant" include any subsequent generation of a plant, whether produced by sexual and/or asexual reproduction.

The terms "transgenic plant" and "transformed plant" refer to equivalent terms of "plant" as described above, where the plant includes a heterologous nucleic acid molecule, heterologous polynucleotide or heterologous polynucleotide construct introduced into the plant by any stable and transient transformation method, e.g., disclosed elsewhere herein or otherwise known in the art. Such transgenic plants and transformed plants also refer, for example, to the plant into which the heterologous nucleic acid molecule, heterologous polynucleotide or heterologous polynucleotide construct has been first introduced, as well as any progeny plants thereof that include the heterologous nucleic acid molecule, heterologous polynucleotide or heterologous polynucleotide construct.

In the disclosure, the use of the term "DNA" or "RNA" is not intended to limit the disclosure to polynucleotide molecules including DNA or RNA. One of ordinary skill in the art will recognize that the methods and compositions of the disclosure include polynucleotide molecules composed of deoxyribonucleotides (i.e., DNA), ribonucleotides (i.e., RNA), or a combination of ribonucleotides and deoxyribonucleotides. Such deoxyribonucleotides and ribonucleotides include naturally occurring molecules and synthetic analogs, including but not limited to: nucleotide analogs or modified backbone residues or linkages, which are synthetic, naturally occurring and non-naturally occurring, which have similar binding properties as the reference nucleic acid, and which are metabolized in a manner similar to the reference nucleotide. Examples of such analogs include, but are not limited to: phosphorothioates, phosphoramidates, methylphosphonates, chiral-methylphosphonates, 2-O-methyl ribonucleotides, peptide-nucleic acids (PNAs). The polynucleotide molecules of the disclosure further inlcude all forms of polynucleotide molecules, including but not limited to: single-stranded forms, double-stranded forms, hairpins, stem-loop structures, etc. In addition, those of ordinary skill in the art should understand that, the nucleotide sequences disclosed herein further include complementary sequences of the exemplary nucleotide sequences.

In the disclosure, the "stringent conditions" mentioned above may be any one of: low stringency conditions, medium stringency conditions, and high stringency conditions. "Low stringency conditions" include, for example, the conditions of: 5×SSC, 5×Denhardt's solution, 0.5% SDS, 50% formamide, and 32°C. "Moderately stringent conditions" include, for example, the conditions of: 5×SSC, 5×Denhardt's solution, 0.5% SDS, 50% formamide, and 42°C. "High stringent conditions" include, for example, the conditions of: 5×SSC, 5×Denhardt's solution, 0.5% SDS, 50% formamide, and 50°C. Among the above conditions, the higher the temperature, the more efficiently a DNA having high homology can be expected to be obtained. Factors that affect hybridization stringency include: temperature, probe concentration, probe length, ionic strength, time, salt concentration, and other factors. Those skilled in the art can achieve the same stringent conditions by appropriately selecting these factors.

**Table 1: Protein and gene sequences of T9αH (or T9H), TOT, TS, T5αH, T13αH, T2αH, T7βH, TAT and TBT**

| Abbreviation | Name | Protein SEQ ID NO: /Uniprot NO: | Gene SEQ ID NO: /NCBINO: |
|---|---|---|---|
| T9αH (or T9H) | Taxane 9α-hydroxylase | 1-14 | 15-28 |
| TOT | Taxol oxetanase (taxane C4-C20 oxetanase) | 29-52 | 53-76 |
| TS | Taxadiene synthase | Q41594, Q93YA3, Q9FT37, A6XA82, B5KRE1, B5KRE3, B5KRE4, B5KRE5, B5KRE6, B5KRE7, B5KRE8, B5KRE9, B5KRF0, B5KRF1, B5KRF2, I6PH40, I6PH46, I6PIQ4, I6PLG0, I6PPZ8, I6PQ08, I6PSS6, I6PST4, I6PST6, L7T9P4, Q29VH9, Q2PRN4, Q67DV5, Q67DX4, Q6SA60, Q6TBY0, W5UFR6 | U48796-AF326519, AJ320538, AY007207, DQ092389, EU107120/ EU107121, EU107122, EU107123, EU107124, EU107125, EU107126, EU107127, EU107128, EU107129, EU107130, EU107131, JQ618980, JQ618979/ JQ618995, JQ618958/ JQ618962/ JQ618963/ JQ618964/ JQ618964/ JQ618965/ JQ618966/ JQ618967/ JQ618969/ JQ618970/ JQ618971/ JQ618972/ JQ618973, JQ618961, JQ618976, JQ618959/ JQ618974/ JQ618975/ JQ618992, JQ618989, JQ618994, KC188793, KC188793, DQ305407, AY365032, AY364469, AY461450, AY424738, KF878108 |
| T5αH | Taxane 5α-hydroxylase | Q6WG30, I6PGZ3, I6PIK2, I6PGY8, I6PGZ7, I6PLA5, I6PPW9, I6PSN0, I6PGY5, I6PGZ1, I6PGZ9, I6PL97, I6PLB3, I6PPV8, I6PPW4, I6PPW7, I6PGZ5, I6PIK4, I6PIK6, I6PIK8, I6PLA3, I6PPW1, I6PSM3, I6PIL0, I6PL94, I6PLA0, I6PPV2, I6PPV5, I6PSM5, I6PSM7, I6PSM8, I6PSN2 | AY289209/ AY741375, JQ618865, JQ618853, JQ618855, JQ618875, JQ618872, JQ618881, JQ618874, JQ618850, JQ618860, JQ618880, JQ618857, JQ618882, JQ618861, JQ618871/ JQ618878, JQ618876, JQ618870, JQ618858, JQ618863, JQ618868, JQ618867, JQ618866, JQ618854, JQ618873, JQ618852, JQ618862, JQ618851, JQ618856, JQ618859, JQ618864/ JQ618877, JQ618869, JQ618879 |
| T13αH | Taxane 13α-hydroxylase | Q8W4T9, Q5BU48, Q56GD5 | AY056019, AY866412, AY959321 |
| T2αH | Taxane 2α-hydroxylase | Q6JD68, Q5S1U2 | AY518383, AY789508 |
| T7βH | Taxane 7β-hydroxylase | Q6JTJ0, H9BII3 | AY307951, JQ029683 |
| TAT | Taxane 5α-acetyl transferase | Q9M6F0, Q8S9G6, Q5Y9C6 | AF190130, AY078285,AY628434 |
| TBT | Taxane 2α-O-benzoyl transferase | Q9FPW3, Q6B7R0, Q1X858, Q1X7Q0, H9BII2 | AF297618, AY675557/ AY864799, AY970522/ AY970523, AY972076, JQ029682 |

Note: The protein SEQ ID NOs of the identified genes in Table 1 are all derived from Uniprot.

The protein and gene sequences of T9αH and TOT provided in Table 1 are derived from the genus *Taxus.*

**Table 2: Similarity of the amino acid sequence of T9αH bioenzyme**

| Name of bioenzyme | SEQ ID NO: | Similarity to T9αH1 |
|---|---|---|
| T9αH1 (or T9H1) | 1 | 100% |
| T9αH2 (or T9H2) | 2 | 83.86% |
| T9αH3 (or T9H3) | 3 | 67.27% |
| T9αH4 (or T9H4) | 4 | 99.60% |
| T9αH5 (orT9H5) | 5 | 67.47% |
| T9αH6 (or T9H6) | 6 | 99.60% |
| T9αH7 (or T9H7) | 7 | 67.66% |
| T9αH8 (or T9H8) | 8 | 99.00% |
| T9αH9 (or T9H9) | 9 | 67.27% |
| T9αH10 (or T9H10) | 10 | 65.67% |
| T9αH11 (or T9H11) | 11 | 65.07% |
| T9αH12 (or T9H12) | 12 | 99.00% |
| T9αH13 (orT9H13) | 13 | 63.75% |
| T9αH14 (or T9H14) | 14 | 63.15% |

**Table 3: Similarity of the nucleotide sequence of T9αH bioenzyme**

| Name of gene | SEQ ID NO: | Similarity to T9αH1 |
|---|---|---|
| T9αH1 (or T9H1) | 15 | 100% |
| T9αH2 (or T9H2) | 16 | 88.47% |
| T9αH3 (or T9H3) | 17 | 77.54% |
| T9αH4 (or T9H4) | 18 | 99.80% |
| T9αH5 (or T9H5) | 19 | 77.71% |
| T9αH6 (or T9H6) | 20 | 99.34% |
| T9αH7 (or T9H7) | 21 | 77.86% |
| T9αH8 (or T9H8) | 22 | 99.34% |
| T9αH9 (or T9H9) | 23 | 77.64% |
| T9αH10 (or T9H10) | 24 | 75.84% |
| T9αH11 (or T9H11) | 25 | 75.83% |
| T9αH12 (or T9H12) | 26 | 99.14% |
| T9αH13 (orT9H13) | 27 | 75.48% |
| T9αH14 (or T9H14) | 28 | 75.83% |

**Table 4: Similarity of the amino acid sequence of TOT bioenzyme**

| Name of bioenzyme | SEQ ID NO: | Similarity to TOT1 |
|---|---|---|
| TOT1 | 29 | 100% |
| TOT2 | 30 | 94.08% |
| TOT3 | 31 | 96.45% |
| TOT4 | 32 | 95.46% |
| TOT5 | 33 | 94.78% |
| TOT6 | 34 | 93.69% |
| TOT7 | 35 | 93.89% |
| TOT8 | 36 | 94.38% |
| TOT9 | 37 | 94.18% |
| TOT10 | 38 | 93.89% |
| TOT11 | 39 | 94.38% |
| TOT12 | 40 | 96.59% |
| TOT13 | 41 | 93.78% |
| TOT14 | 42 | 97.44% |
| TOT15 | 43 | 95.07% |
| TOT16 | 44 | 97.39% |
| TOT17 | 45 | 94.98% |
| TOT18 | 46 | 94.28% |
| TOT19 | 47 | 67.47% |
| TOT20 | 48 | 94.18% |
| TOT21 | 49 | 97.44% |
| TOT22 | 50 | 97.39% |
| TOT23 | 51 | 93.49% |
| TOT24 | 52 | 63.64% |

**Table 5: Similarity of the nucleotide sequence of TOT bioenzyme**

| Name of bioenzymes | SEQ ID NO: | Similarity to TOT1 |
|---|---|---|
| TOT1 | 53 | 100% |
| TOT2 | 54 | 96.21% |
| TOT3 | 55 | 97.84% |
| TOT4 | 56 | 97.06% |
| TOT5 | 57 | 96.73% |
| TOT6 | 58 | 96.14% |
| TOT7 | 59 | 96.14% |
| TOT8 | 60 | 96.73% |
| TOT9 | 61 | 96.60% |
| TOT10 | 62 | 96.08% |
| TOT11 | 63 | 96.46% |
| TOT12 | 64 | 97.87% |
| TOT13 | 65 | 96.01% |
| TOT14 | 66 | 98.10% |
| TOT15 | 67 | 96.66% |
| TOT16 | 68 | 98.07% |
| TOT17 | 69 | 96.60% |
| TOT18 | 70 | 96.14% |
| TOT19 | 71 | 77.56% |
| TOT20 | 72 | 96.07% |
| TOT21 | 73 | 98.30% |
| TOT22 | 74 | 98.27% |
| TOT23 | 75 | 96.08% |
| TOT24 | 76 | 74.85% |

**Table 6: Amino acids, and their names, abbreviations**

| Name | Three letters abbreviation | One letter abbreviation |
|---|---|---|
| Glycine | Gly | G |
| Alanine | Ala | A |
| Valine | Val | V |
| Leucine | Leu | L |
| Isoleucine | Ile | I |
| Proline | Pro | P |
| Phenylalanine | Phe | F |
| Tyrosine | Tyr | Y |
| Tryptophan | Trp | W |
| Serine | Ser | S |
| Threonine | Thr | T |
| Cystine | Cys | C |
| Methionine | Met | M |
| Asparagine | Asn | N |
| Glutamine | Gln | Q |
| Asparticacid | Asp | D |
| Glutamicacid | Glu | E |
| Lysine | Lys | K |
| Arginine | Arg | R |
| Histidine | His | H |

The reagents, instruments or biological materials used in the disclosure are commercial available.

The disclosure will be further described below in conjunction with Examples:

### Example 1: Cloning of Taxane 9α Hydroxylase T9αH Gene

### 1. cDNA acquisition

*Taxus wallichiana var. mairei* was preserved in the laboratory. After collecting young leaves, they were immediately quick-frozen in liquid nitrogen. Some samples were taken and ground in a mortar. 100 mg of the grounded sample was added to a 1.5 mL centrifuge tube, and lysis buffer was added to extract RNA (Plant Total RNA Isolation Kit Plus, FOREGENE, China). The mRNA was reverse transcribed into cDNA by reverse transcription kit (HiScript III 1st Strand cDNA Synthesis Kit, Vazyme, China).

### 2. Cloning of Taxane 9α Hydroxylase T9αH

The genome of *Taxus wallichiana var. mairei* was analyzed, and the amino acid sequence and nucleotide sequence of the T9αH reading frame are represented by SEQ ID NO: 1 (its homologous sequences are respectively represented by SEQ ID NOs: 2-14 in the sequence listing) and SEQ ID NO: 15 (its homologous sequences are respectively represented by SEQ ID NOs: 16-28 in the sequence listing), respectively.

The amino acid sequence of T9αH reading frame (SEQ ID NO: 1):

MDSLSFLKSMEAKFGQVIHRDQSSSTALLSLAFTAALAIFLVLLFRFKSRPSTNFPPGNFGFPFIGETIQ
FLRALRSESPHMFFDERLKKFGRVFKTSLTGHPTAVFCGPAGNRFIYSNEHKLVQSSGPNSFVKLVG
QQSIVTKTGEEHRIFRGVLNEFLGPHALQSYTPKMSSKIQENINKHWKGKDEVNMLPSIRQLVFSISS
SLFFDINDEDQQEQLKTLLETILVGTLSVPLDIPGSNFRKALRARSKLDEILSRLIESRRKDMRSGIAST
SKNLLSVLLAFKDERGNPLTDTEILDNFSFMLHASYDTTVSPTVCIFKLLSANPECYEKVVQEQLGIL
GNKKDGEEICWNDLKAMKYTWQAAQETMRLFPPAFGSFRKVIADIHHDGYIIPKGWKAMVTNYST
SRKEEYFDEPDKFKPSRFGDGKYVAPYTFLPFGAGIRICPGWEFAKLEMLLFIHHFVKNFSGYLPLDT
KEKISGDPFPPLPKNGFPIKLFPRT.

The nucleotide sequence of T9αH reading frame (SEQ ID NO: 15):

ATGGATTCCTTAAGTTTTCTAAAAAGCATGGAAGCGAAATTCGGCCAAGTCATACACCGGGATC
AGTCTTCCAGTACTGCTCTTCTGTCCCTCGCATTCACAGCTGCTCTTGCCATTTTTCTTGTGTTGC
TCTTTCGATTTAAAAGCCGGCCCTCTACTAATTTCCCTCCAGGAAATTTTGGCTTCCCTTTCATTG
GAGAGACGATACAGTTCTTGCGGGCACTTCGATCAGAATCGCCTCATATGTTTTTTGATGAGAG
ATTGAAGAAATTTGGGCGTGTATTCAAGACGTCATTAACTGGGCATCCCACAGCTGTGTTCTGC
GGGCCTGCGGGAAACCGGTTTATTTACTCGAATGAGCACAAGCTGGTGCAGTCGTCTGGGCCCA
ACTCCTTCGTCAAACTGGTTGGGCAGCAATCCATCGTGACCAAAACAGGAGAGGAGCACCGCAT
CTTTCGTGGTGTCCTGAACGAGTTTCTGGGGCCTCATGCCTTACAGAGTTATACGCCTAAAATGA
GTTCCAAAATCCAGGAGAATATCAATAAGCATTGGAAGGGTAAAGATGAAGTGAACATGCTTC
CTTCGATAAGACAGCTCGTCTTCTCCATTTCAAGCAGCTTGTTTTTTGATATTAATGATGAGGAT
CAACAGGAACAACTTAAAACTCTTTTAGAAACTATTCTTGTTGGAACTTTGTCGGTTCCCCTCGA
CATTCCAGGATCTAATTTTCGTAAAGCTCTTCGGGCGCGTTCCAAGCTGGATGAAATTCTGTCTC
GTTTAATCGAAAGCAGAAGAAAAGATATGCGTTCTGGGATAGCTTCTACCAGTAAAAATCTACT
GTCGGTGCTGCTCGCCTTCAAAGATGAAAGAGGGAATCCATTGACGGACACGGAGATCCTCGAC
AACTTTTCTTTTATGCTTCACGCCTCATACGACACCACCGTTTCGCCCACGGTTTGTATATTTAAG
CTGCTCTCCGCCAATCCAGAATGCTATGAAAAAGTAGTTCAAGAACAATTGGGAATACTTGGCA
ATAAAAAGGACGGTGAAGAAATCTGTTGGAACGATCTGAAAGCTATGAAATATACATGGCAAG

CAGCTCAAGAAACAATGAGGCTTTTCCCTCCAGCGTTTGGATCATTTCGCAAGGTCATCGCCGA
TATTCATCATGATGGCTATATAATTCCCAAAGGATGGAAAGCTATGGTGACAAATTACAGTACA
AGTAGGAAAGAAGAGTACTTCGATGAACCAGACAAATTCAAGCCTTCAAGATTTGGGGATGGA
AAGTATGTGGCTCCGTACACGTTCTTACCTTTCGGGGCAGGAATACGCATATGCCCAGGATGGG
AGTTCGCTAAGTTGGAGATGTTACTGTTCATCCATCATTTTGTCAAAAATTTCAGCGGATACCTC
CCACTTGACACCAAGGAAAAGATTTCCGGAGATCCATTCCCTCCTCTCCCCAAAAATGGATTTC
CCATTAAACTATTTCCCAGAACCTAA.

Based on the nucleotide sequence of T9αH gene, primers P1 and P2 for amplifying T9αH gene were designed. The primers included a part of the sequence of tobacco expression vector pEAQ-HT. The T9αH gene was amplified through PCR by using the cDNA of leaves of *Taxus wallichiana var. mairei* as a template. The PCR product was recovered by gel excision, and then recombined with the linear pEAQ-HT vector digested with RruI and XhoI. The ClonExpress one-step cloning kit (Novagen) was used for cloning and sequencing. The positive recombinant plasmid was named pEAQ-HT-T9αH.

Primer sequences are shown in the table below.

**Table 7: Primer sequences**

| Name | SEQ ID NO: | Sequences |
|---|---|---|
| P1 | 77 | 5'-gtatattctgcccaaattcgcgaATGGATTCCTTAAGTTTTCTAAAAAGCATG-3' |
| P2 | 78 | 5'-tgaaaccagagttaaaggcctcgagTTAGGTTCTGGGAAATAGTTTAATGGGAAATC-3' |

Note: The lowercase sequences are the vector sequences, and the uppercase sequences are the T9αH specific primer sequences.

### Example 2: Cloning of Taxane C4-C20 Oxetanase TOT Gene

### 1. cDNA acquisition

Taxus wallichiana var. mairei was preserved in the laboratory. After collecting young leaves, they were immediately quick-frozen in liquid nitrogen. Some samples were taken and ground in a mortar. 100 mg of the grounded sample was added to a 1.5 mL centrifuge tube, and lysis buffer was added to extract RNA (Plant Total RNA Isolation Kit Plus, Foregene, China). The mRNA was reverse transcribed into cDNA by reverse transcription kit (HiScript III 1st Strand cDNA Synthesis Kit, Vazyme, China).

### 2. Cloning of Taxane C4-C20 Oxetanase TOT

The genome of *Taxus wallichiana var. mairei* was analyzed, and the amino acid sequence and nucleotide sequence of the TOT reading frame were represented by SEQ ID NO: 31 (its homologous sequences are respectively represented by SEQ ID NOs: 29, 30, 32-52 in the sequence listing) and SEQ ID NO: 55 (its homologous sequences are respectively represented by SEQ ID NOs: 53, 54, 56-76 in the sequence listing), respectively.

The sequence of SEQ ID NO: 31 is as follows:

MVHVLHVVKMDRVREIFNGSSGSPAGIPHSVITAGVGAIIIILLSLLLLRRSSKRGDSSHPPGNSGLPFI
GETLSFTKAFKSNTLAEFFEERVKKFGNVFKISIIGPPTVVMCGNEGNRFIFANEEKLMHLSWSGRYA
KILGGESVSMKRGDDHRSVRAAFAGFLSSASLPVYISKMSAQIQDHINEKWKGKDVIAVVPLVKELV
FNVSYNLFFSINDSEELHRLHKIFETIVEGHLSMPIDLPGFTFHRALQGRAKLKKVFSSLIERRRSDLSS
GLASANQDLISVLLTYKDDRGYTMTHDELLDNFLSLLEGSYDSINSPMACIFKLLYDNPECYEKVVQ
EQLGILSGKKEGQEISWKDLRSMKYTWQVLQETLRLYTQVAGIFRKAMTDIHYDGHTIPKGWQLL
WANQTTHLNDKYFSEPEKFMPSRFDEEGNNMIPYSFVPFGGGRRMCPGWEFGKMEILLFVHHFVKT
FSGFTPIDPNEKITGNPFPHLPANGFLIKPILRS.

The sequence of SEQ ID NO: 55 is as follows:

ATGGTTCATGTGTTGCACGTAGTGAAAATGGATAGAGTTAGAGAAATATTTAATGGAAGTTCAG
GTTCTCCAGCTGGTATTCCCCACAGTGTGATCACAGCCGGTGTGGGTGCCATAATAATAATTCTT
CTGTCACTACTGCTCCTCCGCCGTTCTAGTAAACGGGGCGACTCCTCTCATCCTCCTGGAATTC
AGGCCTTCCATTCATTGGGGAGACATTATCATTCACCAAGGCTTTAAATCGAACACGCTGGCC
GAATTTTTGAGGAGAGGGTGAAGAAATTCGGGAATGTATTTAAGATTTCAATAATCGGGCCTC
CCACAGTGGTAATGTGCGGCAATGAGGGAAACCGGTTTATTTCGCCAACGAGGAGAAGCTGAT
GCACCTGTCGTGGTCCGGTCGATATGCGAAAATCCTTGGTGGGGAATCCGTTTCCATGAAGAGG
GGAGATGATCATCGCAGTGTACGTGCCGCATTCGCAGGGTTTTGAGCTCTGCATCGCTGCCTGT
TTACATAAGTAAAATGAGTGCACAGATCCAAGATCATATCAACGAAAAATGGAAAGGAAAAGA
TGTAATTGCTGTAGTTCCTCTGGTAAAGGAGCTCGTCTTCAACGTTTCCTACAACTTGTTTTCAG
CATAAATGATAGCGAGGAACTGCATCGATTGCATAAGATTTCGAAACTATTGTGGAGGGACAT
CTTTCCATGCCGATAGACCTTCCCGGATTCACCTTTCATAGAGCACTTCAGGGAAGGGCGAAGC
TCAAGAAAGTTTCTCTTCTTTAATAGAAAGGAGAAGAAGCGATCTGAGCTCCGGATTGGCATC
TGCTAATCAGGATCTCATTTCTGTTTACTCACCTACAAAGATGATAGGGGGTATACAATGACCC
ACGACGAGCTCCTCGACAACTTTCTTTCCCTTCTTGAAGGCTCCTATGATTCCATCAATTCACCA
ATGGCCTGCATTTTTAAGCTTTTGTATGACAATCCAGAATGCTATGAAAAAGTAGTTCAAGAGC
AATTGGGGATACTTTCTGGTAAGAAGGAAGGACAAGAAATCTCGTGGAAGGATCTGAGATCCA
TGAAATACACATGGCAAGTACTTCAGGAAACGCTACGACTGTATACTCAAGTTGCTGGAATATT
TCGCAAAGCCATGACTGACATTCATTATGATGGTCACACCATTCCCAAAGGGTGGCAACTTCTTT

GGGCAAACCAAACTACACATCTGAACGACAAATATTTCAGTGAGCCTGAAAAATTCATGCCTTC
CAGATTCGATGAAGAAGGAAACAATATGATTCCTTACTCATTCGTACCATTTGGAGGAGGGCGG
CGGATGTGTCCAGGTTGGGAATTCGGAAAGATGGAGATCTTACTCTTTGTCCATCACTTTGTTAA
AACGTTCAGTGGCTTTACCCCAATTGATCCGAACGAAAAAATTACTGGGAATCCTTTTCCTCATC
TCCCTGCCAATGGATTTCTAATAAAACCTATTCTCAGATCCTAA.

Based on the nucleotide sequence of the TOT gene, primers P3 and P4 for amplifying the TOT gene were designed. The primers included a part of the sequence of the tobacco expression vector pEAQ-HT. The TOT gene was amplified through PCR by using the cDNA of leaves of *Taxus wallichiana var. mairei* as a template. The PCR product was recovered by gel excision, and then recombined with the linear pEAQ-HT vector digested with RruI and XhoI. The ClonExpress one-step cloning kit (Novagen) was used for cloning and sequencing. The positive recombinant plasmid was named pEAQ-HT-TOT3.

The primer sequences are shown in the table below.

**Table 8: Primer sequences**

| Name | SEQ ID NO: | Sequences |
|---|---|---|
| P3 | 79 | 5'-gtatattctgcccaaattcgcgaATGGTTCATGTGTTGCACGT-3' |
| P4 | 80 | 5'-accagagttaaaggcctcgagTTAGGATCTGAGAATAGGTTTTATTAGAAATCCATT-3' |

Note: The lowercase sequences are the vector sequences, and the uppercase sequences are the TOT-specific primer sequences.

### Example 3: Functional Verification of T9αH and TOT Genes Involved in the Synthesis of Baccatin III in Tobacco

The constructed expression vector pEAQ-HT-T9αH was transferred into Agrobacterium GV3101 to obtain the GV3101/pEAQ-HT-T9αH transgenic strain. The positive single clone was picked and inoculated into LB medium (including 50 µg/mL kanamycin and 25 µg/mL rifampicin), then culturing at 28°C for 24 h; the cultured bacterial solution was taken in 10 mL LB medium (including 50 µg/mL kanamycin and 25 µg/mL rifampicin) at a ratio of 1:100, then culturing at 28°C overnight to an OD₆₀₀ value of 0.8-1.0; the resuspension MMA (10 mM MES, 10 mM MgCl₂, 150 µM acetosyringone) was added until reaching the final OD₆₀₀ of 0.8-1.0, then allowing to stand at room temperature for 1-2 h.

The constructed expression vector pEAQ-HT-TOT was transferred into Agrobacterium GV3101 to obtain the GV3101/pEAQ-HT-TOT3 transgenic strain. The positive single clone was picked and inoculated into LB medium (including 50 µg/mL kanamycin and 25 µg/mL rifampicin), then culturing at 28°C for 24 h; the cultured bacterial solution was taken in 10 mL LB medium (including 50 µg/mL kanamycin and 25 µg/mL rifampicin) at a ratio of 1:100, then culturing at 28°C overnight to an OD₆₀₀ value of 0.8-1.0; the resuspension MMA (10 mM MES, 10 mM MgCl₂, 150 µM acetosyringone) was added until reaching the final OD₆₀₀ of 0.8-1.0, then allowing to stand at room temperature for 1-2 h.

In the same manner, CV3101 Agrobacterium including genes such as TS (U48796), TAT (AF190130 or AY628434, preferably AY628434), T5αH (AY289209), T13αH (AY056019), T2αH (AY518383), T7βH (AY307951), TBT (AF297618), etc. were cultured and resuspended in MMA to an OD₆₀₀ value of 0.8-1.0, and then respectively mixed with Agrobacterium including T9αH and Agrobacterium including TOT in a ratio of 1:1, and Agrobacterium including fluorescent protein GFP is used as a control.

The resuspended Agrobacterium combination was injected into the back of 4-6 week-old leaves of *Nicotiana benthamiana* by a 1 mL syringe without a needle. After drying under light for 1-2 hours, the leaves were transferred to a dark place to culture for 24 hours, and then transferred to normal light for culturing. Five days after the injection of Agrobacterium, the samples were collected, and the leaves injected with GFP were used as controls.

The tobacco sample was ground with a grinder, and the sample powder was transferred to a 5 mL centrifuge tube. After freeze-drying, 1 mL of methanol was added, and ultrasonic extraction was performed for 30 min. After centrifugation at 13,000 rpm for 15 min, the supernatant was transferred to a new centrifuge tube, then centrifuging at 13,000 rpm for 15 min. 200 µL was taken out to put into a sample injection bottle for detection in LC-MS.

The results show that when T9αH and TOT were co-expressed with 7 known genes in the baccatin III biosynthetic pathway, a new product peak 7 could be detected, which has the same mass (m/z 609[M+Na] ⁺) and retention time as baccatin III (7) (Group 1 in Fig. 2). Tandem mass spectrometry (MS/MS) analysis further demonstrated that the new product 7 is baccatin III ( Fig. 3 ).

We further investigated whether these 9 genes are the minimal gene combination for the synthesis of baccatin III in tobacco. As shown in Fig. 2, when any one of the nine genes in the co-expression system was missing, baccatin III could not be detected (Groups 2-10 in Fig. 2). The results show that these 9 genes constitute the core components of baccatin III biosynthesis.In addition, for a long time T10βH has been considered to be an essential gene for baccatin III biosynthesis. The results of the disclosure show that in the absence of T10βH, baccatin III can still be synthesized (Figs. 2-3).

In addition, 10-deacetylbaccatin III-10-β-O-acetyl transferase (DBAT) is considered to be the key enzyme for C10 acylation in baccatin III. The research results of the disclosure show that in the absence of DBAT, baccatin III can still be synthesized (Figs. 2-3), indicating that TAT can replace its function in tobacco.

### Example 4. Constructing the synthetic Pathway of Baccatin III in Yeast to Achieve Heterologous Production

The T9αH (SEQ ID NO: 1) and TOT (SEQ ID NO: 31) genes, as well as TS (U48796), TAT (AY628434), T5αH (AY289209), T13αH (AY056019), T2αH (AY518383), T7βH (AY307951) and TBT (AF297618) genes, were constructed into the expression vector pESC-LEU. The expression frame was amplified by using primers including yeast genome integration sites, and these genes were integrated into the yeast genome by using PEG/lithium acetate transformation to obtain the cpt10.1 strain.

The cpt10.1 strain was inoculated into 2-3 mL of YPD (including 2% glucose) to incubate overnight at 30°C and 200 rpm, then inoculating into 20 mL of YPD (including 2% glucose) at an initial ratio of 0.1 OD₆₀₀ to culture for 2 days, centrifuging at 4000 rpm for 10 minutes. After removing the supernatant, 20 mL of sterile water was added to resuspend, then centrifuging at 4000 rpm for 10 minutes. After removing the supernatant, the bacterial body was resuspended in 20 mL of YPL (including 2% galactose) to incubate at 30°C and 200 rpm for 6-7 days, and the wild-type yeast was used as a control.

After completion of the culturing, an equal volume of ethyl acetate was added to shake and extract at low temperature overnight, then transferring to a 50 mL centrifuge tube, centrifuging at 4000 rpm for 10 minutes, transferring the upper organic phase to a new 50 mL centrifuge tube. After performing vacuum spin dry, 200 µ L of methanol was added to re-dissolve, then transferring 100 µ L to an injection bottle for LC-MS detection.

The results show that a new product peak 7 can be detected in the yeast strain cpt10.1 including 9 genes involved in the baccatin III biosynthetic pathway, and the peak 7 product has the same retention time as baccatin III (Fig. 4).

Only the preferred embodiments of the disclosure are described above. It should be pointed out that for ordinary technicians in this technical field, several improvements and modifications can be made without departing from the principle of the disclosure. These improvements and modifications should also be considered to be within the protection scope of the disclosure.

## Claims

1. A bioenzyme composition, **characterized in that** the bioenzyme composition comprises taxane 9α-hydroxylase (T9αH) and taxane C4-C20 oxetanase (TOT);
the amino acid sequence of T9αH comprises at least one of the following (a1) to (a4):
(a1) an amino acid sequence represented by SEQ ID NO: 1;
(a2) a fusion amino acid sequence obtained by connecting a tag to N-terminus and/or C-terminus of the amino acid sequence represented by SEQ ID NO: 1;
(a3) an amino acid sequence having the same function obtained by substitution and/or deletion and/or addition of one or more amino acid residues in the amino acid sequence represented by SEQ ID NO: 1; and
(a4) an amino acid sequence having at least 60% identity with the amino acid sequence of SEQ ID NO: 1 and having the same function; and
the amino acid sequence of the TOT comprises at least one of the following (b1) to (b4):
(b1) an amino acid sequence represented by SEQ ID NO: 29;
(b2) a fusion amino acid sequence obtained by connecting a tag to N-terminus and/or C-terminus of the amino acid sequence represented by SEQ ID NO: 29;
(b3) an amino acid sequence having the same function obtained by substitution and/or deletion and/or addition of one or more amino acid residues in the amino acid sequence represented by SEQ ID NO: 29; and
(b4) an amino acid sequence having at least 60% identity with the amino acid sequence of SEQ ID NO: 29 and having the same function.

2. A bioenzyme composition according to claim 1, **characterized in that** in (a4), the amino acid sequence having at least 60% identity with and the same function as the amino acid sequence represented by SEQ ID NO: 1 comprises at least one of the amino acid sequences represented by SEQ ID NOs: 2-14; and/or
in (b4), the amino acid sequence having at least 60% identity with and the same function as the amino acid sequence represented by SEQ ID NO: 29 comprises at least one of the amino acid sequences represented by SEQ ID NOs: 30-52.

3. The bioenzyme composition according to claim 1 or 2, **characterized in that** the bioenzyme composition further comprises at least one of: taxadiene synthase (TS), taxane 5α-hydroxylase (T5αH), taxane 13α-hydroxylase (T13αH), taxane 2α-hydroxylase (T2αH), taxane 7β-hydroxylase (T7βH), taxane 5α-acetyl transferase (TAT), and taxane 2α-O-benzoyl transferase (TBT).

4. A bioenzyme composition according to any one of claims 1 to 3, **characterized in that** the bioenzyme composition further comprises at least one of: phenylalanyl-CoA ligase (PCL), phenylalanine aminomutase (PAM), baccatin III 3-amino-3-phenylpropanoyl transferase (BAPT), cytochrome P450 hydroxylase (T2'αH), 3'-N-debenzoyl-2'-deoxytaxol-N-benzoyl transferase (DBTNPT), and geranylgeranyl pyrophosphate synthase (GGPPS).

5. A nucleic acid molecule composition encoding the bioenzyme composition according to any one of claims 1 to 4, **characterized in that** it comprises a nucleic acid molecule encoding T9αH and a nucleic acid molecule encoding TOT;
the nucleotide sequence of the nucleic acid molecule encoding T9αH comprises at least one of the following sequences:
(c1) a nucleotide sequence represented by SEQ ID NO: 15;
(c2) a complementary sequence, a degenerate sequence or a homologous sequence of the nucleotide sequence represented by SEQ ID NO: 15, wherein the homologous sequence is a nucleotide sequence having at least 75% identity with the nucleotide sequence represented by SEQ ID NO: 15; and
(c3) a nucleotide sequence hybridizing with the nucleotide sequence as shown in (c1) or (c2) under stringent conditions and being capable of encoding a protein with the same function; and
the nucleotide sequence of the nucleic acid molecule encoding POT comprises at least one of the following sequences:
(d1) a nucleotide sequence represented by SEQ ID NO: 53;
(d2) a complementary sequence, a degenerate sequence or a homologous sequence of the nucleotide sequence represented by SEQ ID NO: 53, wherein the homologous sequence is a nucleotide sequence having at least 74% identity with the nucleotide sequence represented by SEQ ID NO: 53; and
(d3) a nucleotide sequence hybridizing with the nucleotide sequence as shown in (d1) or (d2) under stringent conditions and being capable of encoding a protein with the same function.

6. A nucleic acid molecule composition according to claim 5, **characterized in that** in (c2), the homologous sequence comprises at least one of the nucleotide sequences represented by SEQ ID NOs: 16-28; and/or
in (d2), the homologous sequence comprises at least one of the nucleotide sequences represented by SEQ ID NOs: 54-76.

7. The nucleic acid molecule composition according to claim 5 or 6, **characterized in that** the nucleic acid molecule composition further comprises a nucleic acid molecule encoding at least one of the following bioenzymes: taxadiene synthase (TS), taxane 5α-hydroxylase (T5αH), taxane 13α-hydroxylase (T13αH), taxane 2α-hydroxylase (T2αH), taxane 7β-hydroxylase (T7βH), taxane 5α-acetyl transferase (TAT), taxane 2α-O-benzoyl transferase (TBT).

8. A nucleic acid molecule composition according to any one of claims 5 to 7, **characterized in that** the nucleic acid molecule composition further comprises a nucleic acid molecule encoding at least one of: a phenylalanyl-CoA ligase (PCL), a phenylalanine aminomutase (PAM), a baccatin III 3-amino-3-phenylpropanoyl transferase (BAPT), a cytochrome P450 hydroxylase (T2'αH), a 3'-N-debenzoyl-2'-deoxytaxol-N-benzoyl transferase (DBTNPT), and a geranylgeranyl pyrophosphate synthase (GGPPS).

9. A biomaterial, **characterized in that** the biomaterial is any one of the following (e1) to (e3):
(e1) an expression cassette comprising the nucleic acid molecule composition according to any one of claims 5 to 8, or a composition thereof;
(e2) a vector or a composition thereof, comprising the nucleic acid molecule composition according to any one of claims 5 to 8, or the expression cassette of (e1) or a composition thereof; and
(e3) a host cell or a composition thereof, comprising at least one of: the nucleic acid molecule composition according to any one of claims 5 to 8, the expression cassette of (e1) or a composition thereof, and the vector of (e2) or a composition thereof.

10. The biological material according to claim 9, **characterized in that** (e1) is an expression cassette in which all nucleic acid molecules are expressed, or a composition of multiple expression cassettes in which all nucleic acid molecules are co-expressed;
(e2) is a vector in which all nucleic acid molecules are expressed, or a composition of multiple vectors in which all nucleic acid molecules are co-expressed; and
(e3) is a host cell in which all nucleic acid molecules are expressed, or a composition of multiple host cells in which all nucleic acid molecules are co-expressed through co-culture and/or multi-stage culture.

11. A method for producing a host cell, **characterized in that** the host cell is transformed by using at least one of: the nucleic acid molecule combination according to any one of claims 5 to 8, and the expression cassettes or a composition thereof, and the vectors or a composition thereof, in the biological material according to claim 9 or 10.

12. A method for producing a plant or plant cell, **characterized in that** the plant or plant cell is transformed by using at least one of: the nucleic acid molecule combination according to any one of claims 5 to 8, and the expression cassettes or a composition thereof, and the vectors or a composition thereof, in the biological material according to claim 9 or 10.

13. A method for producing a bioenzyme composition according to any one of claims 1 to 4, **characterized in that** a host cell or a composition of host cells is transformed by using at least one of: the nucleic acid molecule combination according to any one of claims 5 to 8, the expression cassettes or a composition thereof, and the vectors or a composition thereof, in the biological material according to claim 9 or 10, so that the host cell or a combination host cells produces the bioenzyme composition.

14. Use of any one of the bioenzyme composition according to any one of claims 1 to 4, the nucleic acid molecule composition according to any one of claims 5 to 8, and the biomaterial according to claim 9 or 10 in the synthesis of baccatin III and/or an intermediate thereof.

15. The use according to claim 14, **characterized in that** the baccatin III intermediate comprises a compound represented by the following formula II and/or formula III: 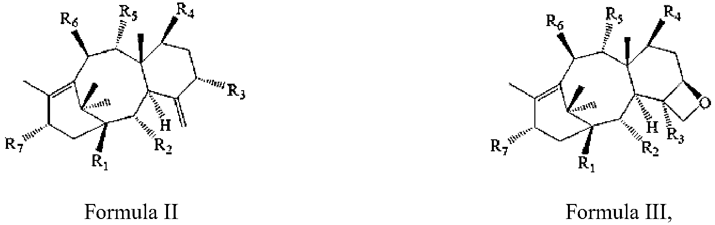 wherein R₁, R₃ and R₄ are independently selected from H, -OH and -OAc; R₂ and R₆ are selected from H, -OH, -OAc and -OBz; and R₅ and R₇ are selected from H, -OH, =O and -OAc.

16. A method for synthesizing baccatin III and/or an intermediate thereof, **characterized in that** under the action of the bioenzyme composition according to any one of claims 1 to 4 and/or the bioenzyme composition produced by the method according to claim 13, the precursor of baccatin III and/or an intermediate thereof is used as a substrate to catalyze hydroxylation at C9 position of taxane ring skeleton and formation of C4-C20 oxetane of the taxane ring skeleton, thereby synthesizing baccatin III and/or an intermediate thereof; and/or
transforming a host cell by using the nucleic acid molecule composition according to any one of claims 5 to 8 or any one of the biological materials (e1)-(e2) according to claim 9 or 10, so that the host cell produce a bioenzyme composition; wherein the host cell comprises at least one of: microbial cell, plant cell, animal cell and algae cell; under the action of the produced bioenzyme composition, the precursor of baccatin III and/or an intermediate thereof is used as a substrate to catalyze hydroxylation of C9 position of taxane ring skeleton and formation of C4-C20 oxetane of the taxane ring skeleton, thereby synthesizing baccatin III and/or an intermediate thereof.

17. The method according to claim 16, **characterized in that** it further comprises any one of the following reactions (f1)-(f3):
(f1) hydroxylation of at least one carbon position of C1, C2, *C5,* C7, C10 and C13 of the taxane ring backbone;
(f2) acylation of at least one hydroxyl group of C2, C5 and C10; and
(f3) ketonization of C9 hydroxyl.

18. The method according to claim 16 or 17, **characterized in that** under the action of a bioenzyme combination comprising at least T2αH, T5αH, T7βH, T9αH, T13αH, TOT, TAT and TBT, baccatin III is synthesized by using taxadiene as a substrate.

19. The method according to claim 18, **characterized in that** the taxadiene as a substrate is synthesized under the action of TS enzyme.

20. Use of any one of the bioenzyme composition according to any one of claims 1 to 4, the nucleic acid molecule composition according to any one of claims 5 to 8, or the biological material according to claim 9 or 10 in the synthesis of a taxane compound and/or an intermediate thereof.

21. A method for synthesizing a taxane compound and/or an intermediate thereof, **characterized in that** it comprises the following steps:
synthesizing baccatin III by the method according to any one of claims 16 to 19; and
using baccatin III as a substrate, and further catalyzing to synthesize the taxane compound and/or an intermediate thereof.

22. The method according to claim 21, **characterized in that** the taxane compound comprises at least one of:
paclitaxel and a derivative thereof, taxusin and a derivative thereof, docetaxel and a derivative thereof, cabazitaxel and a derivative thereof; and
the intermediate of the taxane compound comprises at least one of: β-phenylalanyl baccatin III and 3'-N-debenzoyl paclitaxel.

23. A method for manufacturing a commercial product, **characterized in that** it comprises: obtaining a genetically modified plant or a plant part thereof comprising the nucleic acid molecule composition according to any one of claims 5 to 8, and manufacturing the commercial product from the plant or a plant part thereof, wherein the commercial product is a crude extract, a raw material medicine, and/or a pharmaceutical formulation comprising at least one selected from the group consisting of: baccatin III and a derivative thereof, paclitaxel and a derivative thereof, taxusin and a derivative thereof, docetaxel and a derivative thereof, and cabazitaxel and a derivative thereof.

24. A product generated by a method according to any one of claims 16 to 19 and 21 to 23, **characterized in that** the product comprises at least one of the following ingredients: bacatine III and a derivative thereof, paclitaxel and a derivative thereof, taxusin and a derivative thereof, docetaxel and a derivative thereof, and cabazitaxel and a derivative thereof.
